# EUROPEAN PATENT APPLICATION

(11) **EP 2 680 005 A1**
(43) Date of publication of application: **01.01.2014**
(21) Application number: 12174085.6
(22) Date of filing: 28.06.2012
(51) Int. Cl.: G01N 33/58, G01N 33/92, C07C 57/18, C07C 215/24, C07J 9/00

(54) **Lipid analysis**

(71) Applicant: Rheinische Friedrich-Wilhelms-Universität Bonn, 53113 Bonn (DE)
(72) Inventor: Thiele, Christoph, 53127 Bonn (DE)
(74) Representative: Viering, Jentschura & Partner

(57) **Abstract**

The present invention relates to a method for the detection of a lipid comprising reacting an alkyne-modified lipid derivative and an azido-modified coumarin compound, purifying the resulting compound using chromatography and detecting the reaction product. In a further aspect the present invention provides new alkyne lipid derivatives, new methods of alkyne lipid synthesis, new uses of such compounds and new methods employing these compounds.

## Description

### FIELD OF THE INVENTION

The present invention lies in the field of chemical synthesis, analytics, cell biology, and lipids and relates to novel lipid compounds and novel synthetic routes of synthesis of lipid compounds which can be coupled with detectable labels. The labeled compounds are suitable for lipid analysis, in particular for accurate analysis of lipid metabolism.

### BACKGROUND OF THE INVENTION

The study of metabolic overload diseases has become an important focus of biomedical research, driven by the need to understand the consequences of over-caloric westernized diet. Since metabolic overload usually leads to obesity, studies of fatty acid metabolism are a central aspect of current metabolism research. For that, detailed quantitative information about fatty acid metabolism from all available model systems, such as purified proteins, cultivated cell lines, primary cells, isolated organs and whole organisms is needed. While the need for these data is steadily increasing, we face a concomitant decrease of the accessibility and acceptance of a major technology used to obtain these data in the last 60 years (Kornberg A and Pricer WE, 1953. J Biol Chem 204, 329-343), namely the use of radiolabeled fatty acids for monitoring fatty acid metabolism. A typical experiment using this technology comprises metabolic labeling of living cells or organisms, lipid extraction, lipid separation by TLC and detection of metabolites by autoradiography on X-ray films or scintillation counting (Rock CO and Jackowski S, 1985. J Biol Chem 260, 12720-12724). This sensitive, specific and quantitative method allows parallel comparative analysis of fatty acid incorporation into a broad range of lipid classes. The major drawback of this method is the use of radioactive compounds as such. It requires specialized laboratories and official permissions, and causes significant costs for both purchase of substances and disposal of radioactive waste. Furthermore, many researchers are reluctant to use radiolabeled compounds because of safety concerns. Another drawback is the limited sensitivity of experiments using radioactive fatty acids. The relevant isotopes, ³H and ¹⁴C, have moderate or low specific activities and require long exposure times in order to obtain a meaningful result.

Click-chemistry allows the sensitive and specific detection of compounds containing azido groups or terminal alkynes (Kolb HC, Finn MG, and Sharpless KB, 2001. Angew Chem Int Ed Engl 40, 2004-2021). Both can be integrated into fatty acids without major disturbance of the structure of the hydrophobic hydrocarbon chains. Click-labeled precursors have already been proposed to replace radioactive molecules in metabolic labeling experiments, including amino acids (Beatty KE et al., 2005. J Am Chem Soc 127, 14150-14151; Dieterich DC et al., 2006. PNAS 103, 9482-9487), nucleotides (Salic A and Mitchison TJ, 2008. PNAS 105, 2415-2420) or lipids (Hannoush RN and Sun J, 2010. Nat Chem Biol 6, 498-506). Monitoring protein lipidation using click-labeled fatty acids has been decribed (Hang HC et al., 2007. J Am Chem Soc 129, 2744-2745; Kostiuk MA et al., 2008. FASEB J 22, 721-732; Martin BR and Cravatt BF, 2009. Nat Methods 6, 135-138). However, the use of click-labeled fatty acids to monitor cellular lipid metabolism has however been hampered by the lack of existing protocols that allow sensitive detection of click-labeled lipids after TLC analysis. The present invention solves this problem by use of a fluorogenic click-reaction combined with an optimized protocol for the detection reaction, TLC separation, and fluorescence detection.

### SUMMARY OF THE INVENTION

In a first aspect, the present invention relates to a method for the detection of a lipid comprising a) reacting in a solvent at least one lipid derivatized with at least one terminal alkyne group with at least one coumarin compound derivatized with at least one terminal azido group in the presence of catalytic amounts of Cu(I) to form a triazolyl coumarin compound derivative of the at least one lipid, b) purifying the triazolyl coumarin compound derivative of the at least one lipid by chromatography, and c) detecting the triazolyl coumarin compound derivative of the at least one lipid, wherein i) after purifying the triazolyl coumarin compound derivative of the at least one lipid and before its detection the triazolyl coumarin compound derivative of the at least one lipid is treated with a base and/or ii) the reacting step comprises providing the at least one coumarin derivative compound derivatized with at least one terminal azido group in excess over the at least one lipid derivatized with at least one terminal alkyne group and evaporating the solvent to concentrate the reaction mix and achieve complete reaction of the at least one lipid derivatized with at least one terminal alkyne group with the at least one coumarin derivative compound derivatized with at least one terminal azido group to form a triazolyl coumarin compound derivative of the at least one lipid.

In some embodiments of the method of the invention, the lipid is selected from the group consisting of saturated fatty acids, unsaturated fatty acids, glycerolipids, glycerophospholipids, lysophosphoglycerolipids, sphingolipids, sterol lipids, prenol lipids, saccharolipids, carotinoids, waxes, and polyketides.

In further embodiments of the method, the coumarin compound is derivatized with an azido group, the azido coumarin derivative optionally being a 3-azido hydroxy coumarin.

In certain embodiments, the coumarin derivative is selected from the group consisting of 3-, 4-, 5-, 6-, 7-, and 8-hydroxy azido coumarin.

In various embodiments, reaction step a) comprises the addition of a Cu(I) salt or complex.

In some embodiments, reaction step a) comprises the addition of a Cu(I) salt or complex, wherein the Cu(I) salt or complex is selected from the group consisting of CuI, CuBr, CuCl, CuOTf*C₆H₆, [Cu(NCCH₃)₄], and Cu[acetonitrile]₄BF₄, Cu[acetonitrile]₄PF₆, or combinations thereof.

In certain embodiments, the reaction step a) comprises evaporating the solvent of the reaction mixture to increase the concentration of the at least one lipid derivatized with at least one terminal alkyne group to a concentration range of 5-40 µM.

In various embodiments, the reaction step a) comprises evaporating the solvent of the reaction mixture to increase the concentration of the at least one coumarin derivative compound derivatized with at least one terminal azido group to a concentration range of 50-5000 µM, optionally in the range of 300-1000 µM.

In some embodiments, the reaction step a) comprises providing the at least one coumarin derivative compound derivatized with at least one terminal azido group with a concentration range of 40-100 µM, optionally 60 µM.

In various embodiments, the chromatography of step b) is selected from the group consisting of HPLC, gel chromatography, TLC, and SMART.

In some embodiments, the chromatography of step b) is TLC and the samples are first developed in a solvent system comprising or consisting of CHCl₃/MeOH/H₂O/AcOH, for example in a volume ratio of 65/25/4/1, and then in a solvent system comprising/consisting of hexane/ethyl acetate, for example in a volume ratio of 1/1.

In various embodiments, the detection in step c) is fluorescence detection.

In certain embodiments, the base of step i) is selected from the group of volatile and non-fluorescent bases.

In various embodiments, the base of step i) is selected from the group of bases consisting of N,N-diisopropylethylamine, triethylamine, trimethylamine, monoethylamine (MEA), diethylamine (DEA), triethylamine (TEA), monoisopropylamine, monoisopropylamine (MIPA), diisopropylamine (DIPA), dimethylaminopropylamine (DEAPA), dimethyl dipropylene Triamine (DMAPAPA), 3-Isopropoxypropylamine (IPOPA), 3-methoxypropylamine (MOPA), ethylmethylamine (EMA), tetramethylpropylenediamine (TMPDA), sec-butylamine (B2A), aminopropyldiethanolamine (APDEA), dimethyl isopropyl amine (DMIPA), dimethylethylamine (DMEA), and diethylhydroxylamine (DEHA) or mixtures thereof.

In further embodiments, the lipid may be selected from the group consisting of oleate, palmitate, cholesterol, cholesterol ester, cardiolipin, diacylglycerol, phosphatidylcholine, phosphatidylethanolamine, phosphatidylglycerol, lysophosphoglycerolipids, phosphatidylinositol, phosphatidylserine, triacylglycerol, and sphinganin.

In some embodiments, the method comprises a step of extracting the at least one lipid derivatized with at least one terminal alkyne group from a cell. This step may be performed before the reacting step a). The cell may have been incubated with the at least one lipid derivatized with at least one terminal alkyne group. In one embodiment, the at least one lipid derivative is selected from the group consisting of an terminal alkyne cholesterol derivative, omega alkyne oleate, omega alkyne palmitate, omega alkyne sphinganin, omega alkyne fatty acid, and omega alkyne unsaturated fatty acid.

In another aspect, the present invention relates to a compound having the structural formula: 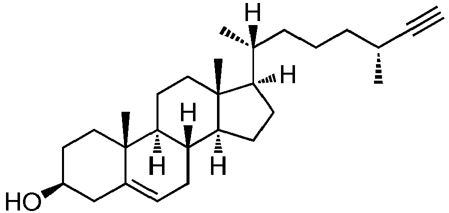 and the salts and/or esters thereof.

In a further aspect, the present invention relates to a compound having the structural formula: 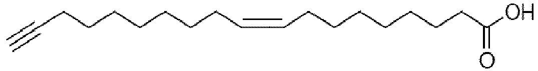 and the salts and/or esters thereof.

In a further aspect, the present invention relates to a compound having the structural formula: 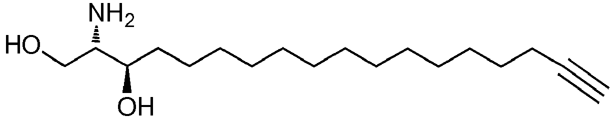 and the salts and/or esters thereof.

In certain aspects, the present invention relates to the product obtained by reacting the above alkyne-derivatized lipids of the invention with a 3,-, 4-, 5,-, 6-, 7-, or 8-hydroxy azido coumarin derivative.

In some embodiments, the alkyne compounds disclosed herein comprise a fluorescent tag which is coupled to the compound via a triazole group formed by reaction of the alkyne group of the compound and an azido moiety bound to a fluorophore.

In another aspect, the present invention relates to the use of a compound disclosed herein in a fluorescence detection assay.

In a further aspect, the present invention relates to the use of a compound disclosed herein for determining the catalytic activity of an enzyme.

In another aspect, the present invention relates to the use of a compound disclosed herein for analyzing the lipid metabolism in a cell, tissue, organ, or organism.

In a further aspect, the present invention relates to the use of a lipid derivative with at least one terminal alkyne group as disclosed herein for synthesis of at least one alkyne lipid derivative.

In another aspect, the present invention relates to a method of synthesis of alkyne lipids derivatives comprising contacting a cell with at least one compound selected from the group consisting of alkyne cholesterol, omega alkyne oleate, omega alkyne palmitate and alkyne sphinganine, cultivating the cell under conditions wherein the at least one compound is subjected to the cell's lipid metabolism, and extracting the alkyne derivatives produced by the cell. In some embodiments, the accordingly synthesized alkyne lipid derivatives are detected using the detection method disclosed herein.

In another aspect, the present invention relates to a method of synthesis of (2S,3R)-2-aminooctadec-17-yn-1,3-diol (alkyne-sphinganine) comprising:
a) reacting THP-protected 1-tetradecyn-14-ol and Garners aldehyde to give *tert-Butyl* (4*S*, 1'*R*)-4-(1'-hydroxypentadec-2'-yn-15-(2-tetrahydropyranyloxy)-yl-2,2-dimethyl-3-oxazolinecarboxylate,
b) reacting the product of step a) with H₂ in the presence of Pd/C to give *tert*-Butyl (4*S*, 1*'R*)-4-(1'-Hydroxypentadec-15-(2-tetrahydropyranyloxy)-yl-2,2-dimethyl-3-oxazolinecarboxylate,
c) reacting the product of step b) with acetic anhydride in the presence of pyridine and DMAP to give *tert*-Butyl (4*S*,1*'R*)-4-(1'-Acetoxypentadec-15-(2-tetrahydropyranyloxy))-yl-2,2-dimethyl-3-oxazolinecarboxylate,
d) reacting the product of step c) with acetone, MeOH, and toluenesulfonic acid to give *tert-Butyl* (4*S*,1*'R*)-4-(1'-Acetoxypentadec-15-hydroxy)-yl-2,2-dimethyl-3-oxazolinecarboxylate,
e) reacting the product of step d) with pyridiniumchlorochromate (PCC) in the presence of molecular sieve powder to give the corresponding aldehyde,
f) reacting the product of step e) with dimethyl (1-diazo-2-oxopropyl)phosphonate (Bestmann-Ohira reagent), potassium carbonate, and MeOH to give *tert-Butyl* (4*S*,1*'R*)-4-(1'-hydroxyhexadec-15-yn)-yl-2,2-dimethyl-3-oxazolinecarboxylate, and
g) reacting the product of step f) in THF with HCl to give (2S,3R)-2-Aminooctadec-17-yn-1,3-diol.

In a further aspect, the present invention relates to a method of synthesis of (25R)-25-ethinyl-26-nor-3b-hydroxycholest-5-en (click-Cholesterol) comprising:
a) reacting (25R)-3b-(tert-Butyldimethylsilyloxy)cholest-5-en-26-ol with pyridiniumchlorochromate (PCC) in the presence of powdered molecular sieves to give (25*R*)-3*b*-(*tert*-Butyldimethylsilyloxy)cholest-5-en-26-al, and
b) reacting the product of step a) with dimethyl-(1-diazo-2-oxopropyl)phosphonate (Bestmann-Ohira reagent), potassium carbonate, MeOH, and THF, followed by the treatment of the resulting product with HCl and THF to give(25R)-25-ethinyl-26-nor-3b-hydroxycholest-5-en.

In a further aspect, the present invention relates to a method of synthesis of a compound having a structural formula CH≡C-(CH₂)ₙ-COOH, wherein n is an integer selected from the group consisting of 1, 2, 3, 4, 5, 6, 7, 8, 9, 10, 11, 12, 13, 14, 15, 16, 17, 18, 19, 20, 21, and 22, comprising:
a) reacting a compound having a formula Br-CH₂-CH₂-(CH₂)ₙ-COOH with KOH to give HO-CH₂-(CH₂)ₙ-COOH,
b) reacting the product of step a) with ROH, H⁺ to give HO-CH₂-(CH₂)ₙ-COO-R, wherein R is selected from the group comprising -CH₃, -CH₂CH₃, -n-propyl, -i-propyl, -n-butyl, -sec-butyl, and -t-butyl,
c) reacting the compound of step b) with pyridinium chlorochromate (PCC) in presence of molecular sieve powder to give O=CH-CH₂-(CH₂)ₙ-COO-R,
d) reacting the compound of step c) with potassium carbonate, dimethyl (1-diazo-2-oxopropyl)phosphonate (Bestmann-Ohira reagent), and methanol to give CH≡C-CH₂-(CH₂)ₙ-COO-R, and
e) subjecting the product of step d) to ester hydrolysis to give CH≡C-CH₂-(CH₂)ₙ-COOH.

In another aspect, the present invention relates to a method of synthesis of omega alkyne unsaturated fatty acids comprising
a) reacting a compound having a formula of HC≡C-(CH₂)ₙ-CH₂-OH with pyridinium chlorochromate (PCC) in presence of molecular sieve powder to give HC≡C-(CH₂)ₙ-CH=O, wherein n is an integer selected from the group consisting of 1, 2, 3, 4, 5, 6, 7, 8, 9, 10, and 11,
b) reacting a compound having a formula of Br-CH₂-(CH₂)ₘ-CH₂-OH with CrO₃/H₂SO₄ to give Br-CH₂-(CH₂)ₘ-COOH, wherein m is an integer selected from the group consisting of 1, 2, 3, 4, 5, 6, 7, 8, 9, and 10,
c) reacting Br-CH₂-(CH₂)ₘ-COOH with triphenylphosphin at a temperature of 80-150°C to give the corresponding bromine salt of the omega triphenylphosphinyl fatty acid derivative, and
d) reacting the product of step c) in dry THF under argon with two equivalents of lithium hexamethylene-disilazane, cooling the reaction mixture to -55°C, adding 1,3-dimethyl-3,4,5,6-tetrahydro-2(1H)-pyrimidinone (DMPU), followed by addition of the product of step a) solubilized in THF to give HC≡C-(CH₂),-CH=CH-(CH₂)ₘ₋COOH.

In a further aspect, the present invention relates to a method of synthesis of omega alkyne unsaturated fatty acids comprising
a) reacting a compound having a formula of HO-CH₂-(CH₂)ₙ-CH₂-OH with propionic acid and toluenesulfonic acid to give CH₃C(=O)O-CH₂-(CH₂)ₙ-CH₂-OH, wherein n is an integer selected from the group consisting of 1, 2, 3, 4, 5, 6, 7, 8, 9, 10, 11, and 12,
b) reacting the compound of step a) with pyridinium chlorochromate (PCC) in the presence of molecular sieve powder to give CH₃C(=O)O-CH₂-(CH₂)ₙ-CH=O,
c) reacting a compound having a formula of Br-CH₂-(CH₂)ₘ-CH₂-OH with CrO₃/H₂SO₄ to give Br-CH₂-(CH₂)ₘ-COOH, wherein m is an integer selected from the group consisting of 1, 2, 3, 4, 5, 6, 7, 8, 9, 10, 11, and 12,
d) reacting Br-CH₂-(CH₂)ₘ-COOH with triphenylphosphin in acetonitrile at a temperature of 80-150°C to give the corresponding bromine salt of the omega triphenylphosphinyl fatty acid derivative,
e) reacting the product of step b) with two equivalents of lithium hexamethylene-disilazane in dry THF under argon, cooling the reaction mix to -55°C, adding 1,3-dimethyl-3,4,5,6-tetrahydro-2(1H)-pyrimidinone (DMPU), followed by addition of the product of step b) solubilized in THF to give CH₃C(=O)O-CH₂-(CH₂)ₙ-CH=CH-(CH₂)ₘ-COOH,
f) reacting the product of step e) with MeOH and acetylchloride to give *cis* HO-CH₂-(CH₂)ₙ-CH=CH-(CH₂)ₘ-COO-CH₃,
g) reacting the compound of step f) with pyridinium chlorochromate (PCC) in DCM in the presence of molecular sieve powder to give *cis* O=CH-(CH₂)ₙ-CH=CH-(CH₂)ₘ-COO-CH₃,
h) reacting the compound of step g) with potassium carbonate, dimethyl (1-diazo-2-oxopropyl)phosphonate (Bestmann-Ohira reagent), and methanol to give *cis* CH=C-(CH₂)ₙ-CH=CH-(CH₂)ₘ-COO-CH₃, and
i) reacting the product of step h) with KOH, MeOH, H₂O and HCl to give *cis* CH=C-(CH₂)ₙ-CH=CH-(CH₂)ₘ-COOH.

In some embodiments, step f may further comprise purification by chromatography on a AgNCV silica column.

### BRIEF DESCRIPTION OF THE DRAWINGS

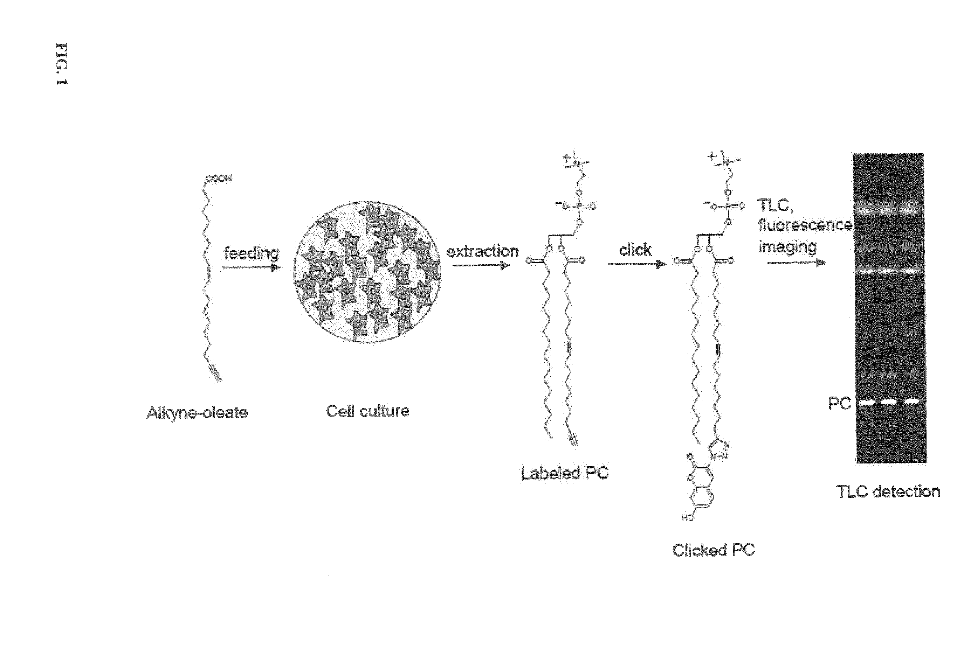

**Figure 1** shows a schematic representation of a typical workflow of the method of the present invention. Alkyne lipid precursors are supplemented to cell culture where they become incorporated **into** cellular lipids, e.g. into phosphatidylcholine (PC). These are extracted and reacted with the fluorogenic dye 3-azido-7-hydroxycoumarin in the presence of copper(I). The fluorescent lipids are then separated on TLC plates and detected by fluorescence imaging.

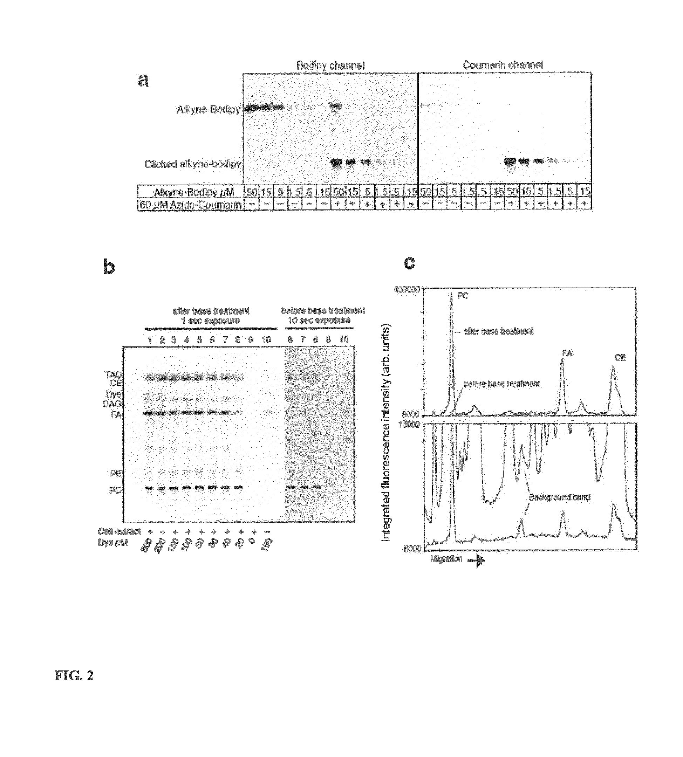

**Figure 2** shows that the click detection reaction is highly efficiency and specificity. (**a**) Alkyne-Bodipy at concentrations as indicated was reacted with either 60 µM 3-azido-7-hydroxycoumarin and 2mM Cu(I)BF₄ (+) or incubated in ethanol for 3h at 42°C (-). The samples were separated by TLC using hexane/ethyl acetate 1/1 as the mobile phase. The dried TLC plate was first imaged with excitation at 485 nm and emission at >520 nm (left panel, Bodipy channel), followed by treatment with 4% Hünig's base in hexane and imaging with excitation at 420 nm and emission at 484-504 nm (right panel, Coumarin channel). (b, c) Influence of dye concentration and base treatment. A172 cells were grown in a 15 cm dish and labeled with 100 µM alkyne-palmitate/alkyne-oleate 1/1 for 2 h. Lipids were extracted and dissolved in 2 ml CHCl₃. 2 µl of this extract were reacted with 2 mM Cu(I)BF₄ and the indicated concentration of 3-azido-7-hydroxycoumarin in 37 µl ethanol/MeCN/CHCl₃ 24/6/7 at 42°C for 3 h. Samples were separated by TLC and imaged **(b)** before (right panel) and after (left panel) treatment with 4%-Hünig's base in hexane for the times indicated. (c) Plot of the integrated fluorescence intensity of lane 6 of panel b (left) and from the corresponding one second exposure before base treatment. The upper panel displays the full intensity range, while the lower shows a magnification of the low intensities. Note the background band that remains unchanged upon base treatment.

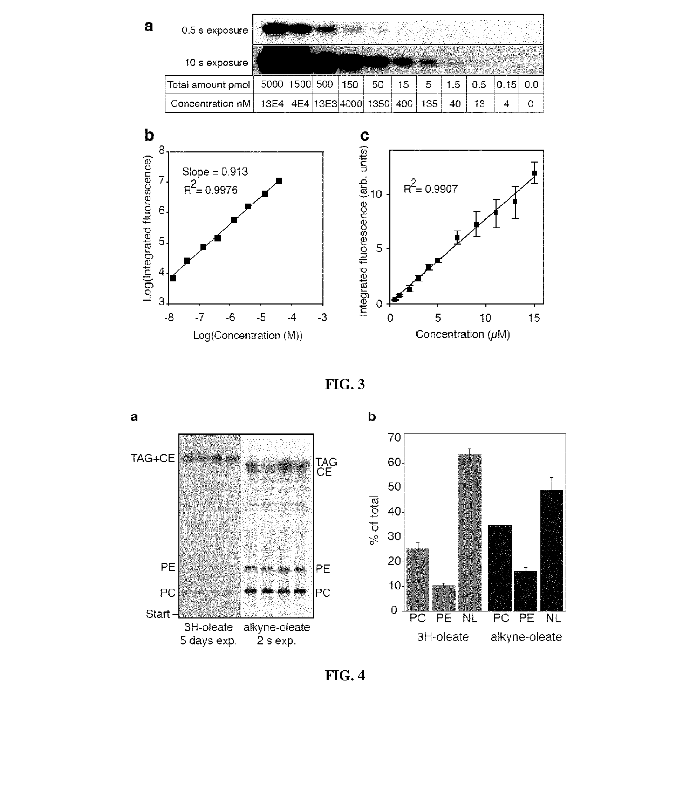

**Figure 3** shows an analysis of the sensitivity and linearity of the detection. The cholesterol ester of **alkyne-oleate** at the indicated concentrations was subjected to detection reaction, TLC separation and fluorescence imaging using the standard protocol. **(a)** Images of the same plate exposed for 0.5 s or 10 s. **(b)** Quantification of integrated fluorescence of **panel a.** Data are displayed in a double log plot. The slope of 0.91 indicates only small deviations from linearity over a concentration range of 3.5 magnitudes. Note that the sample with the highest concentration in panel a was not included in the analysis, because the analyte concentration exceeded the dye concentration. (c) The same experiment was performed with samples at the practically most relevant concentration range of 0.5-15 µM. These data are displayed in a non-logarithmic plot as mean ± SD of three independent experiments.
**Figure 4** shows data of a comparison of ³H-oleate and alkyne-oleate labeling. Freshly isolated mouse hepatocytes were plated on 10 cm dishes in Williams E medium plus 1% FA-free BSA and labeled at 37°C for 10 min with a mixture of 20 µM alkyne-oleate, 20 µM oleate and 3 µCi/ml of ³H-oleate. After a 20 min chase in Williams E medium + 1% FA-free BSA, cells were washed and scraped and lipids were extracted. Equal aliquots (1/4 of total) were either directly applied on TLC, sprayed with scintillant and exposed to X-ray film **(panel a,** left), or subjected to click-reaction, TLC and imaging following the standard protocol **(panel a,** right). Signals were quantified either by scraping, extraction and scintillation counting **(panel b,** ³H-oleate) or by integration of signals form the fluorescence image **(panel b,** alkyne-oleate). All differences between bars are statistically significant with p<0.01 (paired student's T-test). NL: neutral lipids, TAG + CE.

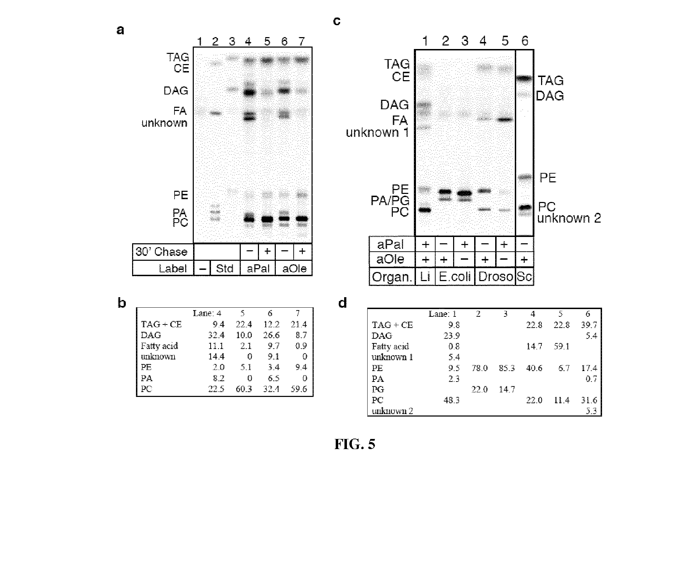

**Figure 5** illustrates typical applications of the compounds of the present invention. **(a+b)** Pulse chase labeling in HuH7 hepatoma cells. Cells (lanes 4-9) were grown in 6 well plates and labeled for 3 min with alkyne fatty acids (aPal, aOle) as indicated, followed by no chase (-) or a 30' chase (+) as indicated. Cells were washed, scraped and subjected to lipid extraction. Lipids extracts were dried and subjected to detection reaction, TLC separation and fluorescence imaging using the standard protocol. Lane 1 represents the background of the click-reaction, lanes 2 and 3 are clicked synthetic standards as indicated at the left of the panel. (b) Quantification of the signals in panel a. All numbers are percent of total of the respective lane. **(c+d)** Application in various different model systems. Mouse liver (Li) was perfused for 5 min with a mixture of 66 µM alkyne-oleate and 33 µM alkyne-pal in Williams medium + 1% FA-free BSA, followed by a 2 min wash in label-free medium, E. coli (E. coli) was grown for 3 h with 20 µM alkyne fatty acids as indicated, single drosophila L3 larvae (Droso) were opened and incubated for 30 min with 50 µM alkyne lipids as indicated. Saccharomyces cerevisiae (Sc) was incubated for 2 h with 50 µM alkyne-oleate. Lipids were extracted and lipid extracts were subjected to detection reaction, TLC separation and fluorescence imaging using the standard protocol. The amounts loaded correspond to 0.1% of one mouse liver (lane 1), 1 ml E. coli culture (lanes 2, 3), one single L3 larva (lanes 4, 5) and 2 ml of yeast culture (lane 6). Note that lanes 1-5 are from one plate, lane 6 is from a separate experiment with different running distances of solvent systems 1 and 2, leading to different positions of lipids on the plate. **(d)** Quantification of the signals in panel c. All numbers are percent of total of the respective lane.

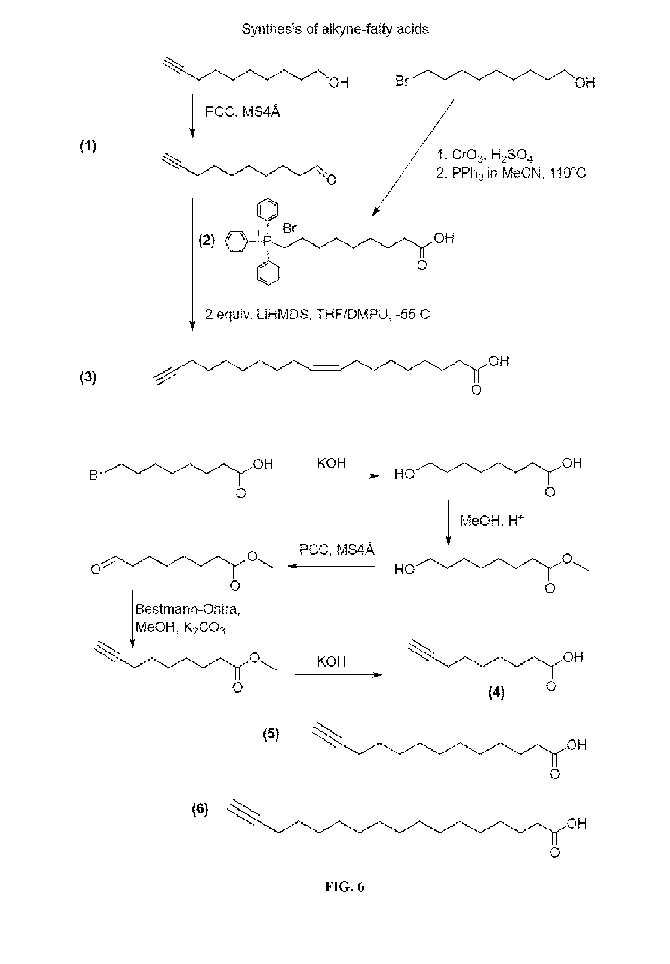

**Figure 6** shows synthesis pathways for click fatty acids and click unsaturated fatty acids.

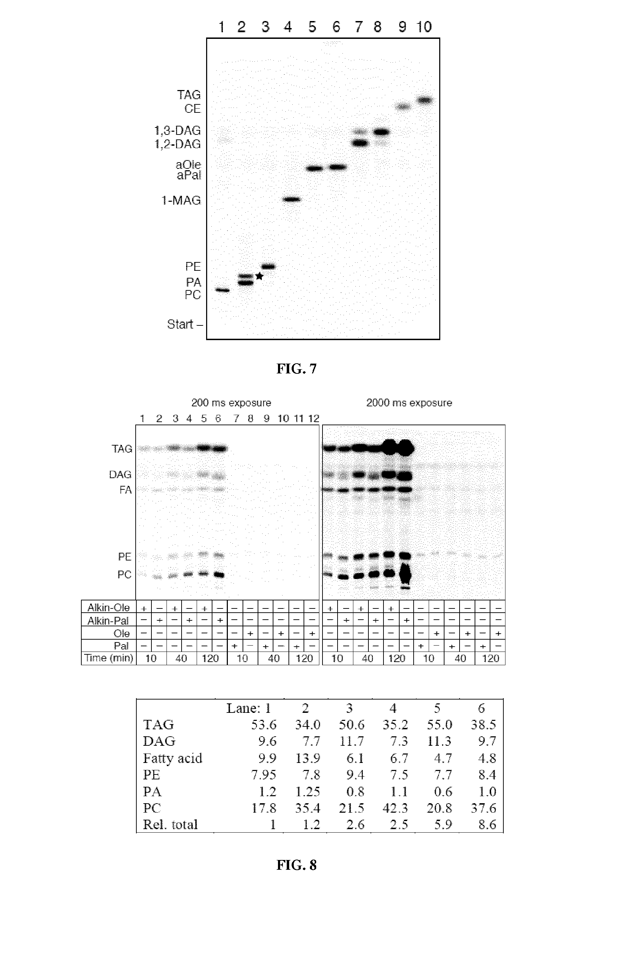

**Figure 7** shows an analysis of the lipid standards used in the experiments disclosed herein. Standard lipids (0.1-0.3 nmol) were subjected to detection reaction, TLC separation and fluorescence imaging using the standard protocol. The asterisk indicates the unnatural 1,3-PA. The lower PA band is the natural 1,2-PA.
**Figure 8** shows an analysis of the hepatocyte fatty acid metabolism. Freshly isolated hepatocytes were incubated in 3 cm dishes with 50 µM alkyne-fatty acids or corresponding unlabeled fatty acids for various times as indicated. Cells were collected and split into two equal aliquots. One aliquot was subjected to lipid extraction followed by detection reaction, TLC separation and fluorescence imaging using the standard protocol with the exception of a higher dye concentration (300 µM). The other aliquot can be used for mass spectrometrical analysis of lipids. (a) Detection of labeled lipids by TLC + fluorescent imaging. (b) Quantification of the signals in panel A. All numbers are percent of total of the respective lane. The row labeled "Rel. total" indicates the sum of the absolute signals, normalized to the value of lane 1.

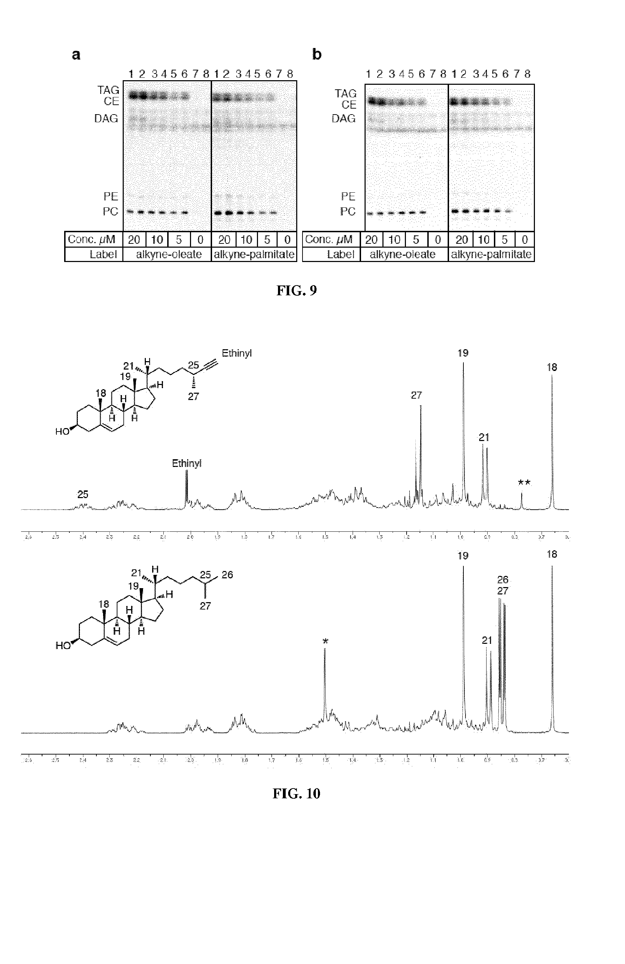

**Figure 9** shows the results of a fatty acid metabolism study, wherein the study was performed in a 96-well format. Therefore, A172 cells were grown in 96-well plates and labeled for 75 min with alkyne fatty acids as indicated. Plates were washed and excess liquid removed by short upside-down centrifugation. (A) Lipids were extracted directly from the dish using CHCl₃/MeOH 1/5, dried and subjected to detection reaction, TLC separation and fluorescence imaging using the standard protocol. (B) Lipids were extracted directly from the dish using the standard click-mix, incubated at 45° for 3h, and subjected to TLC separation and fluorescence imaging as usual. Note that the bands appear qualitatively similar, but show more signal and details in panel A.
**Figure 10** shows a comparison of ¹H-NMR spectra of alkyne-cholesterol (upper panel) and authentic commercial cholesterol (lower panel). Note the loss of the 26-CH₃ signal, the shift of the 27-CH₃ signal and the doublet of the ethinyl-H. The asterisk denotes the water peak in the commercial cholesterol sample, the double asterisk an impurity by remnants of the cleaved TBDMS protecting group. Only the region between 0.6 and 2.6 ppm is shown. The low-field region is identical for both compounds.

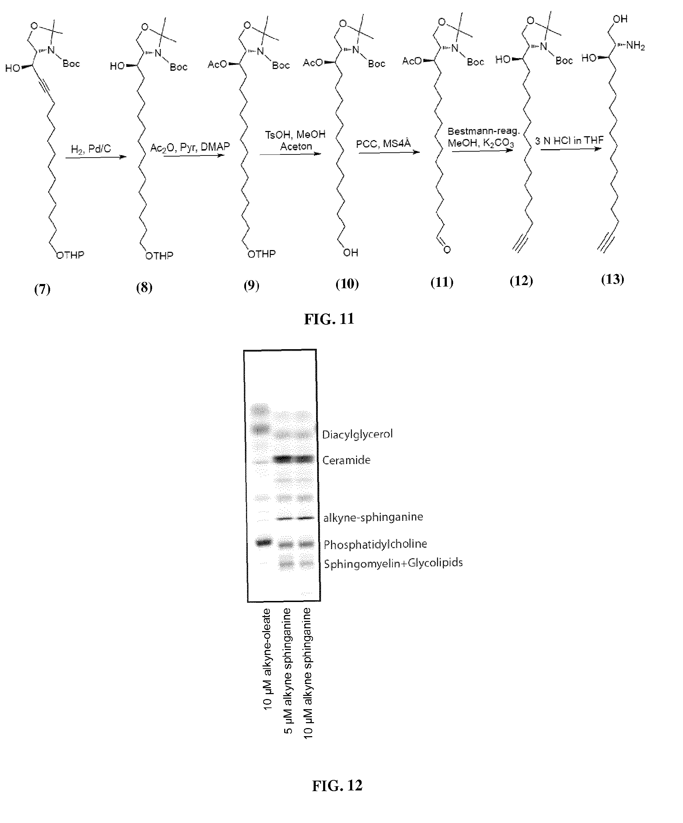

**Figure 11** shows a synthesis pathway for alkyne-sphingine.
**Figure 12** shows the results of an experiment in which A-172 cells were cultivated for 1h in the presence of alkyne-oleate (alkyne-ole) or alkyne-sphinganine as indicated. Afterwards, the cells were collected, lysed, and lipids extracted and click-analyzed according to the standard protocol. As can be seen from the figure, labeling with alkyne-sphinganine results in strong labeling of ceramide and other sphingolipids. Some labeling also occurs in PC, DAG, and other glycerolipids. This is due to the degradation of alkyne-sph via the sphingosine-1-phosphate lyase pathway, which converts parts of alkyne-sph to 15-hexadecynoic acid, which will be incorporated into all glycerolipids.

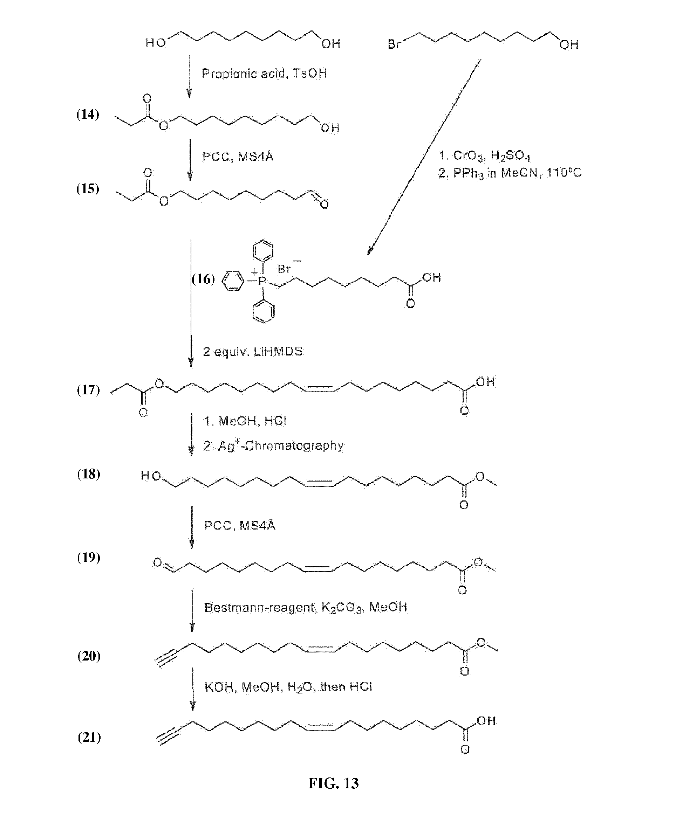

**Figure 13** shows an alternative synthesis pathway for alkyne-oleate.

### DETAILED DESCRIPTION OF THE INVENTION

### Abbreviations:

- CE: cholesterol ester
- CL: cardiolipin
- DAG: diacylglycerol
- FA: fatty acid
- MS: mass spectrometry
- MS/MS: tandem mass spectrometry
- PA: phosphatic acid
- PC: phosphatidylcholine
- PE: phosphatidylethanolamine
- PG: phosphatidylglycerol
- PI: phosphatidylinositol
- PS: phosphatidylserine
- TAG: triacylglycerol
- TBTA: Tris[(1-benzyl-1H-1,2,3-triazol-4-yl)methyl]amin)
- TLC: thin layer chromatography
- aPal; Alkyne-Palmitate: 16-Heptadecynoic acid
- aOle; Alkyne-Oleate: Nonadec-9-cis-en-18-ynoic acid
- alkyne-sphinganine, alkyne-sph: (2S,3R)-2-aminooctadec-17-yn-1,3-diol
- alkyne-cholesterol: (25R)-25-ethinyl-26-nor-3b-hydroxycholest-5-en

The terms used herein have, unless explicitly stated otherwise, the following meanings.

"At least one", as used herein, relates to one or more, in particular 1, 2, 3, 4, 5, 6, 7, 8, 9, 10 or more.

As used herein, the terms "comprises," "comprising," "includes," "including," "has," "having," "contains" or "containing," or any other variation thereof, are intended to cover a non-exclusive inclusion. For example, a composition, a mixture, process, method, article, or apparatus that comprises a list of elements is not necessarily limited to only those elements but may include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

The term "invention" or "present invention" as used herein is a non-limiting term and is not intended to refer to any single embodiment of the particular invention but encompasses all possible embodiments as described in the specification and the claims.

As used herein, the term "about" modifying the quantity of an ingredient or reactant of the invention employed refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term "about" also encompasses amounts that differ due to different equilibrium conditions for a composition resulting from a particular initial mixture. Whether or not modified by the term "about", the claims include equivalents to the quantities. In one embodiment, the term "about" means within 10% of the reported numerical value, preferably within 5% of the reported numerical value.

The term "derivative", as used herein, means an organic compound containing a core structural element similar to that from which it is derived. In particular, in the sense of the present invention, derivatives are compounds which differ from a parent compound due to the presence of additional terminal alkyne and/or azido group. Accordingly, the lipid derivatives differ from the parent compound due to the presence of a terminal alkyne group, whereas coumarin derivatives differ from the parent compound due to the presence of a terminal azido group. In the context of the present invention, a terminal azido group of a coumarin compound is a azido group bound to position 3 or 4 of the coumarin ring system, meaning that the azido group is bound to the 2-pyranone ring of the coumarin ring system, or to position 6 or 7 of the coumarin ring system, meaning that the azido group is bound to the phenyl ring of the coumarin ring system. In some embodiments, the azido group is bound to position 3 or 4 of the coumarin ring system. In various embodiments, the coumarin is bound to position 3 of the coumarin ring system. In certain embodiments, the coumarin is bound to position 4 of the coumarin ring system. In various embodiments, the coumarin is bound to position 6 of the coumarin ring system. In some embodiments, the coumarin is bound to position 7 of the coumarin ring system.

It is understood in the context of the present invention that if a specific compound is mentioned herein also its salts are encompassed by the disclosure of the present invention. Furthermore, also if a salt of a compound is disclosed herein, the compound itself belongs to the disclosure of the present application. For example, the term oleate also encompasses oleic acid and vice versa. Similarly, palmitate also comprises palmitic acid and vice versa.

The term "lipid" as used herein encompasses any known lipid. In particular, the term, as used herein, comprises saturated fatty acids, unsaturated fatty acids, glycerolipids, glycerophospholipids, lysophosphoglycerolipids, sphingolipids, sterol lipids, prenol lipids, saccharolipids, carotenoids, waxes, and polyketides.

As used herein, the term "alkyne" is given its ordinary meaning in the art and refers to a chemical species containing at least one carbon-carbon triple bond (i.e., "-C≡C-").

The term "alkyne group" as used herein, relates to a moiety comprising or consisting of at least one carbon-carbon triple bond (i.e., "-C≡C-"). An alkyne group may consist of a branched or unbranched hydrocarbon group, for example containing 2 to about 24 carbon atoms, and at least one carbon-carbon triple bond. In certain embodiments, the alkyne group consists of 2, 3, 4, 5, 6, 7, 8, 9, or 10 carbon atoms and at least one carbon-carbon triple bond. In various embodiments, the alkyne group is selected from the group consisting of -C≡CH, -C≡C-, 1-propynyl, 2-propynyl, 1-methyl-2-propynyl, 1-n-butynyl, 2-n-butynyl, 3-n-butynyl, 3-methyl-1-butynyl, 1-methyl-2-butynyl, 2-methyl-3-butynyl, 1-pentynyl, 2-pentynyl, 3-pentynyl, and 4-pentynyl. In various embodiments, the alkyne group is-C=CH.

As used herein, the term "terminal alkyne" relates to a moiety comprising or consisting of a carbon-carbon triple bond -C≡C-H, wherein the "-" denotes the attachment point to the compound to which the alkyne is bound.

In some embodiments the alkyne group comprises a terminal alkyne. Accordingly, the alkyne group may consist of a branched or unbranched hydrocarbon group, for example containing 2 to about 24 carbon atoms, and a terminal carbon-carbon triple bond. In certain embodiments, the alkyne group consists of 2, 3, 4, 5, 6, 7, 8, 9, or 10 carbon atoms and a terminal carbon-carbon triple bond. In various embodiments, the alkyne group is selected from the group consisting of -C≡CH, 2-propynyl, 1-methyl-2-propynyl, 3-n-butynyl, 2-methyl-3-butynyl, and 4-pentynyl. In various embodiments, the alkyne group is -C≡CH.

As used herein "triazolyl group" means a 1,2,3-triazole substituent.

The term "azido group" as used herein refers to a -N₃ group.

The present invention relates to click chemistry as disclosed in EP1507769B1 and US 2010/0189660A1, the contents of which are herein incorporated by reference in their entirety.

The basic click-chemistry reaction scheme can be described as follows:

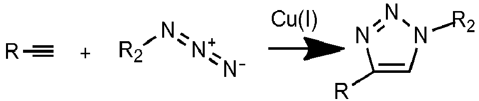

In the context of the present invention R is a lipid, e.g., oleate, palmitate, cholesterol, or sphinganine, and R₂ is a fluorescent label, e.g. a coumarin derivative. Preferably, the R is a lipid and R₂ is a coumarin derivative. The scheme above serves exemplary purposes only and the present invention is not limited thereto. In particular, reaction schemes resulting in structural isomers or stereoisomers of the above product are also intended to be encompassed.

In a first aspect, the present invention relates to a method for the detection of a lipid comprising a) reacting in a solvent at least one lipid derivatized with at least one terminal alkyne group with at least one coumarin derivative compound derivatized with at least one terminal azido group in the presence of catalytic amounts of Cu(I) to form a triazolyl coumarin compound derivative of the at least one lipid, b) purifying the triazolyl coumarin compound derivative of the at least one lipid by chromatography, and c) detecting the triazolyl coumarin compound derivative of the at least one lipid, wherein i) after purifying of the triazolyl coumarin compound derivative of the at least one lipid and before its detection the triazolyl coumarin compound derivative of the at least one lipid the compound is treated with a base and/or ii) the reacting step comprises providing the at least one coumarin derivative compound derivatized with at least one terminal azido group in excess over the at least one lipid derivatized with at least one terminal alkyne group and evaporating the solvent to concentrate the reaction mix and achieve complete reaction of the at least one lipid derivatized with at least one terminal alkyne group with the at least one coumarin derivative compound derivatized with at least one terminal azido group to form a triazolyl coumarin compound derivative of the at least one lipid.

The term "excess" as used herein refers to molecular excess. Preferably, the at least one coumarin derivative compound derivatized with at least one terminal azido group is in 1-1000 fold, for example 10-1000 or 10-500 fold excess over the at least one lipid derivatized with at least one terminal alkyne group.

In some embodiments, the reaction is carried out at 35-50 °C.

Suitable reaction containers are for example Eppendorf tubes equipped with lids. The reaction mix can be filled into these tubes and the tubes put into a heating block set to a temperature falling within the range of 35-50°C. Then, the solvent starts to evaporate and condensates beneath the lid of the tube. Due to the evaporation of the solvent the reaction components are concentrated. After the reaction is completed, the solvent beneath the lid is recovered at the bottom of the tube via centrifugation. This allows for quick resuspension of the product and its processing in the next method step.

In some embodiments, the reacting step comprises evaporating the solvent to concentrate the at least one lipid derivatized with at least one terminal alkyne group to a concentration range of 0.1-500µm, for example 0.2-200 µM or 0.5-40 µM.

In further embodiments, the reaction step comprises evaporating the solvent of the reaction mixture to increase the concentration of the at least one lipid derivatized with at least one terminal alkyne group to a concentration range of 1-500µM, for example 5-40 µM.

In certain embodiments, the reaction step comprises evaporating the solvent of the reaction mixture to increase the concentration of the at least one coumarin derivative compound derivatized with at least one terminal azido group to a concentration range of 50-5000 µM, optionally in the range of 300-1000 µM.

In various embodiments, reaction step a) comprises providing the at least one coumarin derivative compound derivatized with at least one terminal azido group with a concentration range of 1-500µM, for example 40-100 µM, optionally at about 60 µM.

Using excess of the at least one coumarin derivative compound derivatized with at least one terminal azido group over the at least one lipid derivatized with at least one terminal alkyne group is advantageous in order to drive the reaction to completeness.

According to the present invention, completeness means that 80-100% of the total amount of lipid derivatized with at least one terminal alkyne group is reacted to a triazolyl coumarin compound derivative of the at least one lipid. Preferably, 90, 95, 97.5, 99, 99.5, or 100% of the total amount of lipid derivatized with at least one terminal alkyne group is reacted to a triazolyl coumarin compound derivative of the at least one lipid.

A complete reaction of the at least one lipid derivatized with at least one terminal alkyne group is advantageous because it allows for studying even very low amounts of lipid derivative. This is particularly advantageous if cells are exposed to the lipid derivatives of the present invention to study the metabolization of the lipid derivatives. Using the herein described approach, even minor lipid metabolites of the lipid derivatives are detectable. Thus, a highly sensitive lipid derivative detection is provided which allows for an accurate a sensitive lipid analysis, especially lipid metabolism analysis.

The term "purifying", as used herein also comprises the separation of a desired compound from other compounds/components that may for example be present in a sample, mixture or composition, e.g. by chromatography or other suitable separation techniques. Accordingly, as used herein, "purifying" also includes "separating".

Detection techniques that can be employed in the present invention include optical methods, electrochemical methods (voltametry and amperometry techniques), thermal conductivity, mass spectrometry, atomic force microscopy, and radio frequency methods, e.g., multipolar resonance spectroscopy. Illustrative of optical methods, in addition to microscopy, both confocal and non-confocal, are detection of fluorescence, luminescence, chemiluminescence, absorbance, reflectance, transmittance, and birefringence or refractive index, e.g., surface plasmon resonance, ellipsometry, a resonant mirror method, a grating coupler waveguide method or interferometry.

As used herein, the term "chromatography" refers to a process in which a chemical mixture carried by a liquid or gas is separated into components as a result of differential distribution of the chemical entities as they flow around or over a stationary liquid or solid phase.

In some embodiments, the chromatography is liquid chromatography.

As used herein, the term "liquid chromatography" or "LC" means a process of selective retardation of one or more components of a fluid solution as the fluid uniformly percolates through a column of a finely divided substance, or through capillary passageways. The retardation results from the distribution of the components of the mixture between one or more stationary phases and the bulk fluid, (i.e., mobile phase), as this fluid moves relative to the stationary phase(s). Examples of separation techniques which employ "liquid chromatography" include reverse phase liquid chromatography (RPLC), high performance liquid chromatography (HPLC), and turbulent flow liquid chromatography (TFLC) (sometimes known as high turbulence liquid chromatography (HTLC) or high throughput liquid chromatography). In some embodiments, an SPE column may be used in combination with an LC column. For example, a sample may be purified with a TFLC extraction column, followed by additional purification with a HPLC analytical column.

In various embodiments, the chromatography of step b) is selected from the group consisting of HPLC, gel chromatography, turbulent flow liquid chromatography, TLC, and SMART.

As used herein, the term "high performance liquid chromatography" or "HPLC" (sometimes known as "high pressure liquid chromatography") refers to liquid chromatography in which the degree of separation is increased by forcing the mobile phase under pressure through a stationary phase, typically a densely packed column.

As used herein, the term "turbulent flow liquid chromatography" or "TFLC" (sometimes known as high turbulence liquid chromatography or high throughput liquid chromatography) refers to a form of chromatography that utilizes turbulent flow of the material being assayed through the column packing as the basis for performing the separation. TFLC has been applied in the preparation of samples containing two unnamed drugs prior to analysis by mass spectrometry. See, e.g., Zimmer et al., J Chromatography A 854: 23-35 (1999); see also, U.S. Pat. Nos. 5,968,367, 5,919,368, 5,795,469, and 5,772,874, which further explain TFLC. Persons of ordinary skill in the art understand the meaning of "turbulent flow". When fluid flows slowly and smoothly, the flow is called "laminar flow". For example, fluid moving through an HPLC column at low flow rates is laminar. In laminar flow the motion of the particles of fluid is orderly with particles moving generally in straight lines. At faster velocities, the inertia of the water overcomes fluid frictional forces and turbulent flow results. Fluid not in contact with the irregular boundary "outruns" that which is slowed by friction or deflected by an uneven surface. When a fluid is flowing turbulently, it flows in eddies and whirls (or vortices), with more "drag" than when the flow is laminar. Many references are available for assisting in determining when fluid flow is laminar or turbulent (e.g., Turbulent Flow Analysis: Measurement and Prediction, P. S. Bernard & J. M. Wallace, John Wiley & Sons, Inc., (2000); An Introduction to Turbulent Flow, Jean Mathieu & Julian Scott, Cambridge University Press (2001)).

As used herein, the term "solid phase extraction" or "SPE" refers to a process in which a chemical mixture is separated into components as a result of the affinity of components dissolved or suspended in a solution (i.e., mobile phase) for a solid through or around which the solution is passed (i.e., solid phase). In some instances, as the mobile phase passes through or around the solid phase, undesired components of the mobile phase may be retained by the solid phase resulting in a purification of the analyte in the mobile phase. In other instances, the analyte may be retained by the solid phase, allowing undesired components of the mobile phase to pass through or around the solid phase. In these instances, a second mobile phase is then used to elute the retained analyte from the solid phase for further processing or analysis. SPE, including utilization of a turbulent flow liquid chromatography (TFLC) column as an extraction column, may operate via a unitary or mixed mode mechanism. Mixed mode mechanisms utilize ion exchange and hydrophobic retention in the same column; for example, the solid phase of a mixed-mode SPE column may exhibit strong anion exchange and hydrophobic retention; or may exhibit column exhibit strong cation exchange and hydrophobic retention.

As used herein, the term "analytical column" refers to a chromatography column having sufficient chromatographic plates to effect a separation of materials in a sample that elute from the column sufficient to allow a determination of the presence or amount of an analyte. Such columns are often distinguished from "extraction columns", which have the general purpose of separating or extracting retained material from non-retained materials in order to obtain a purified sample for further analysis. In a preferred embodiment the analytical column contains particles of about 4 µm in diameter.

As used herein, the term "on-line" or "inline," for example as used in "on-line automated fashion" or "on-line extraction," refers to a procedure performed without the need for operator intervention. For example, by careful selection of valves and connector plumbing, the solid phase extraction and liquid chromatography columns can be connected as needed such that material is passed from one to the next without the need for any manual steps. In preferred embodiments, the selection of valves and plumbing is controlled by a computer pre-programmed to perform the necessary steps. Most preferably, the chromatography system is also connected in such an on-line fashion to the detector system, e.g., an MS system. Thus, an operator may place a tray of multi-well or multi-tube samples in an autosampler and the remaining operations are performed under computer control, resulting in purification and analysis of all samples selected. In contrast, the term "off-line" as used herein refers to a procedure requiring manual intervention of an operator. Thus, if samples are subjected to precipitation, and the supernatants are then manually loaded into an autosampler, the precipitation and loading steps are off-line from the subsequent steps. In various embodiments of the methods, one or more steps may be performed in an on-line automated fashion.

In certain embodiments, the chromatography is TLC. The skilled person is aware of suitable protocols for developing a TLC based lipid separation. Suitable instructions can be found, e.g., in Touchstone JC, 1995. J Chromatogr B, 671, 169-195 and Sherma and Fried, 2003. Handbook of Thin-Layer Chromatography, 3rd Ed., Marcel Dekker, Inc..

In some embodiments, the chromatography of step b) is TLC and the samples are first developed in CHCl₃/MeOH/H₂O/AcOH 65/25/4/1 and then in hexane/ethyl acetate 1/1. This is particularly suitable in connection with silica gel-based TLC plates.

However, CHCl₃ may be replaced by DCM and/or MeOH may be replaced by other alcohols. Also, THF, dioxane, and other ethers may be employed to achieve an appropriate separation. Furthermore, AcOH may be replaced by other acids. Alternatively, the separation may be carried out under basic conditions using NH3, triethyl amine or other organic amines. The water content of the system may fall within the range of 0 to 20 v/v%.

Alternatively, a hexane/ ethyl acetate (EA) system may be employed. The hexane may also be replaced by other alkanes and EA may be replayed by ethers, chloroform or DCM.

Of course, reverse phase systems may be employed using different solvent combinations which are well known to the skilled person in the art.

In further embodiments, the chromatography is HPLC. In some embodiments the chromatography is gel chromatography.

In various embodiments, the detection in the sense of the present invention is fluorescence detection. In some embodiments, in the method of the present invention the detection in step c) is fluorescence detection.

However, the detection may also be selected from the group consisting of fluorescence detection, luminescence detection, absorbance detection, polarization detection, circular dichroism detection, MS-based detection, chromatography-MS-coupled detection, HPLC-MS-based detection, GC-coupled detection, Gas Chromatography-Mass Spectrometry (GC/MS)-based detection, NMR-based detection, and IR-based detection.

As used herein, the term "gas chromatography" refers to chromatography in which the sample mixture is vaporized and injected into a stream of carrier gas (as hydrogen, nitrogen, or helium) moving through a column containing a stationary phase composed of a liquid or a particulate solid and is separated into its component compounds according to the affinity of the compounds for the stationary phase.

The GC-coupled detection may be selected from the group consisting of flame ionization detector (FID), the thermal conductivity detector (TCD), catalytic combustion detector (CCD), discharge ionization detector (DID), dry electrolytic conductivity detector (DELCD), electron capture detector (ECD), flame photometric detector (FPD), Hall electrolytic conductivity detector (ElCD), helium ionization detector (HID), nitrogen phosphorus detector (NPD), infrared detector (IRD), mass spectrometer (MS), photo-ionization detector (PID), pulsed discharge ionization detector (PDD), and thermionic ionization detector (TID).

As used herein, the term "mass spectrometry" or "MS" refers to an analytical technique to identify compounds by their mass. MS refers to methods of filtering, detecting, and measuring ions based on their mass-to-charge ratio, or "m/z." MS technology generally includes (1) ionizing the compounds to form charged compounds; and (2) detecting the molecular weight of the charged compounds and calculating a mass-to-charge ratio. The compounds may be ionized and detected by any suitable means. A "mass spectrometer" generally includes an ionizer and an ion detector. In general, one or more molecules of interest are ionized, and the ions are subsequently introduced into a mass spectrographic instrument where, due to a combination of magnetic and electric fields, the ions follow a path in space that is dependent upon mass ("m") and charge ("z"). See, e.g., U.S. Pat. No. 6,204,500, entitled "Mass Spectrometry From Surfaces"; U.S. Pat. No. 6,107,623, entitled "Methods and Apparatus for Tandem Mass Spectrometry"; U.S. Pat. No. 6,268,144, entitled "DNA Diagnostics Based On Mass Spectrometry"; U.S. Pat. No. 6,124,137, entitled "Surface-Enhanced Photolabile Attachment And Release For Desorption And Detection Of Analytes"; Wright et al., Prostate Cancer and Prostatic Diseases 2:264-76 (1999); and Merchant and Weinberger, Electrophoresis 21:1164-67 (2000).

As used herein, the term "operating in positive ion mode" refers to those mass spectrometry methods where positive ions are generated and detected. The term "operating in negative ion mode" as used herein, refers to those mass spectrometry methods where negative ions are generated and detected.

As used herein, the term "ionization" or "ionizing" refers to the process of generating an analyte ion having a net electrical charge equal to one or more electron units. Negative ions are those having a net negative charge of one or more electron units, while positive ions are those having a net positive charge of one or more electron units.

In certain embodiments, the detection comprises MS-based detection. MS-based detections may be selected from the group consisting of MS-based on EI, CI, FAB, MALDI, SELDI, ESI, APCI, APPI, FD, and ICP.

As used herein, the term "electron ionization" or "EI" refers to methods in which an analyte of interest in a gaseous or vapor phase interacts with a flow of electrons. Impact of the electrons with the analyte produces analyte ions, which may then be subjected to a mass spectrometry technique.

As used herein, the term "chemical ionization" or "CI" refers to methods in which a reagent gas (e.g. ammonia) is subjected to electron impact, and analyte ions are formed by the interaction of reagent gas ions and analyte molecules.

As used herein, the term "fast atom bombardment" or "FAB" refers to methods in which a beam of high energy atoms (often Xe or Ar) impacts a non-volatile sample, desorbing and ionizing molecules contained in the sample. Test samples are dissolved in a viscous liquid matrix such as glycerol, thioglycerol, m-nitrobenzyl alcohol, 18-crown-6 crown ether, 2-nitrophenyloctyl ether, sulfolane, diethanolamine, and triethanolamine. The choice of an appropriate matrix for a compound or sample is an empirical process.

As used herein, the term "matrix-assisted laser desorption ionization" or "MALDI" refers to methods in which a non-volatile sample is exposed to laser irradiation, which desorbs and ionizes analytes in the sample by various ionization pathways, including photo-ionization, protonation, deprotonation, and cluster decay. For MALDI, the sample is mixed with an energy-absorbing matrix, which facilitates desorption of analyte molecules.

As used herein, the term "surface enhanced laser desorption ionization" or "SELDI" refers to another method in which a non-volatile sample is exposed to laser irradiation, which desorbs and ionizes analytes in the sample by various ionization pathways, including photo-ionization, protonation, deprotonation, and cluster decay. For SELDI, the sample is typically bound to a surface that preferentially retains one or more analytes of interest. As in MALDI, this process may also employ an energy-absorbing material to facilitate ionization.

As used herein, the term "electrospray ionization" or "ESI," refers to methods in which a solution is passed along a short length of capillary tube, to the end of which is applied a high positive or negative electric potential. Solution reaching the end of the tube is vaporized (nebulized) into a jet or spray of very small droplets of solution in solvent vapor. This mist of droplets flows through an evaporation chamber, which is heated slightly to prevent condensation and to evaporate solvent. As the droplets get smaller the electrical surface charge density increases until such time that the natural repulsion between like charges causes ions as well as neutral molecules to be released.

As used herein, the term "atmospheric pressure chemical ionization" or "APCI," refers to mass spectroscopy methods that are similar to ESI; however, APCI produces ions by ion-molecule reactions that occur within a plasma at atmospheric pressure. The plasma is maintained by an electric discharge between the spray capillary and a counter electrode. Then ions are typically extracted into the mass analyzer by use of a set of differentially pumped skimmer stages. A counterflow of dry and preheated N2 gas may be used to improve removal of solvent. The gas-phase ionization in APCI can be more effective than ESI for analyzing less-polar species.

The term "Atmospheric Pressure Photoionization" or "APPI" as used herein refers to the form of mass spectroscopy where the mechanism for the photoionization of molecule M is photon absorption and electron ejection to form the molecular ion M+. Because the photon energy typically is just above the ionization potential, the molecular ion is less susceptible to dissociation. In many cases it may be possible to analyze samples without the need for chromatography, thus saving significant time and expense. In the presence of water vapor or protic solvents, the molecular ion can extract H to form MH+. This tends to occur if M has a high proton affinity. This does not affect quantitation accuracy because the sum of M+ and MH+ is constant. Drug compounds in protic solvents are usually observed as MH+, whereas nonpolar compounds such as naphthalene or testosterone usually form M+. See, e.g., Robb et al., Atmospheric pressure photoionization: An ionization method for liquid chromatography-mass spectrometry. Anal. Chem. 72(15): 3653-59 (2000).

As used herein, the term "inductively coupled plasma" or "ICP" refers to methods in which a sample interacts with a partially ionized gas at a sufficiently high temperature such that most elements are atomized and ionized.

As used herein, the term "field desorption" or "FD" refers to methods in which a non-volatile test sample is placed on an ionization surface, and an intense electric field is used to generate analyte ions.

As used herein, the term "desorption" refers to the removal of an analyte from a surface and/or the entry of an analyte into a gaseous phase.

As used herein, the term "limit of quantification", "limit of quantitation" or "LOQ" refers to the point where measurements become quantitatively meaningful. The analyte response at this LOQ is identifiable, discrete and reproducible with a precision of 20% and an accuracy of 80% to 120%. The upper limit of quantitation refers to the upper quantifiable linear range of analyte response.

As used herein, the term "limit of detection" or "LOD" is the point at which the measured value is larger than the uncertainty associated with it. The LOD is defined arbitrarily as 3 standard deviations (SD) from the zero concentration.

The inventors of the present invention have surprisingly found that the claimed methods, compounds and uses allow for highly efficient, rapid, reliable, and easy lipid detection, especially on the basis of alkyne lipid derivatives. Furthermore, lipid and lipid metabolism analysis can be conveniently and reliably applied in high-throughput applications, as the present invention allows for sufficient miniaturization. Figure 9 demonstrates that the present invention can be adapted to 96-well conditions.

Compared to other approaches, the present invention provides a method of lipid analysis with an improved signal to background ratio and allows for an analysis of even low abundant lipid classes. The applicability of the present invention is universal and thus, the analysis of lipids can be performed in any cell and is not restricted to a certain cell type. Depending on the cell type, of course, the skilled person in the art may choose different alkyne lipid derivatives to monitor the lipid metabolism.

Furthermore, the inventors have found that the terminal alkyne lipid derivatives disclosed herein are metabolized similar to the corresponding natural lipids and that the alkyne derivatives are therefore suitable for monitoring lipid metabolism.

The skilled person may consult any known reference concerning metabolic pathways, e.g. Michal G (Ed.), Biochemical Pathways, Spektr. Verl., 1999, or access known lipid database or metabolic pathway data base, e.g., The LIPID MAPS (LIPID Metabolites And Pathways Strategy; http://www.lipidmaps.org), Lipid Library (http://lipidlibrary.co.uk), Lipid Bank (http://lipidbank.jp), LIPIDAT (http://www.lipidat.chemistry.ohio-state.edu), and Cyberlipids (http://www.cyberlipid.org) and choose a lipid, which, in a derivatized state with a terminal alkyne-group, is suitable for use in the methods of the present invention.

The methods as disclosed herein combine several advantages. First, the sensitivity is higher than that of a ¹⁴C-labeling experiment and at least comparable to that of a ³H-labeling experiment. The lower limit of detection of 0.4 pmol corresponds to 24 pCi of ¹⁴C fatty acid (60 mCi/mmol) or 20 nCi of ³H-fatty acid (50 Ci/mmol). While the former would normally not be detectable on an X-ray film, the latter would require overnight exposure on X-ray film for reliable detection, compared to less than a second for the fluorescence imaging. In theory, undiluted ³H-labeled fatty acid has a lower absolute level of detection than that of the present click method, provided that exposure times of several days or even weeks are accepted. In practice, ³H-fatty acids for cell labeling are rarely used in undiluted form but are diluted with unlabeled material to save money and reduce problems with radiation safety (2 ml of undiluted 50 µM ³H-oleate would contain 5 mCi activity), leading to reduced sensitivity as seen in Figure 4. Second, the excellent linearity of the signal generated by the click procedure allows quantification directly from the image. Furthermore, a quantitative analysis of a TLC plate with 12 lanes and 10 bands per lane can be performed in less than one hour, and can be automated using appropriate software macros. Third, the method is highly versatile and can be adapted to virtually any experimental condition or model organism. Here, the lack of radioactivity is particularly beneficial, because it removes the restrictions that are frequently faced if a complex experimental setup needs to be combined with radioactive work (e.g. missing genetic safety permissions for the isotope lab; missing equipment in the isotope lab, such as complex machines needed for cell sorting, mass spectrometry or automated multiwell plate handling etc.), allowing spontaneous barrier-free experiments. Fourth, the method is more cost-efficient compared to known method using radioactive isotopes. Furthermore, the higher sensitivity allows downscaling of experiments with the concomitant reduction of costs for cell culture media and growth supplements. Fifth, the method saves time and increases productivity. A complete labeling experiment including the quantitative analysis and evaluation is normally finished in one working day. In contrast, the radioactive method needs more time already in the experiment itself because of the typical safety precautions. Once the actual experiment is done, it takes typically 2-6 days for the exposure of the TLC plate, and another day for the scraping-extraction-scintillation counting procedure.

The use of the lipid derivatives of the present invention thus allows to avoid the use of radioisotope labeled compounds. Compared to previous approaches involving coumarin labeled compounds and the detection of the coumarin fluorescence, the detected coumarin fluorescence can be increased by at least 60-fold by employing a base treatment step prior to the detection. Furthermore, the optimized reaction conditions reduce the background signal and increase the quantity of labeled and detectable compound. Due to the optimized conditions allowing for increased sensitivity and the reduction of the reaction volume, the method of the present invention may be employed in miniaturized configuration. For example, the method of the present invention may be employed in a high-throughput screen. Moreover, the improved lipid detection provides a linear and quantifiable readout. Using the methods disclosed herein, dose-response relations may be determined. As radioisotope detection is omitted, the compounds, methods and uses disclosed herein can be combined with further compounds and methods without being limited by radioisotope safety concerns.

In some embodiments of the methods of the invention, the lipid of the lipid derivative is selected from the group consisting of saturated fatty acids, unsaturated fatty acids, glycerolipids, glycerophospholipids, lysophosphoglycerolipids, sphingolipids, sterol lipids, prenol lipids, saccharolipids, carotenoids, waxes, and polyketides.

A classification of lipids is disclosed in Fahy E et al., 2005. J Lipid Res, 46, 839-862, the contents of which, in particular all lipids disclosed therein, are herein incorporated by reference in their entirety.

Specific examples of lipids are dodecanoic acid, 1-hexadecanoyl-2-(9Z-octadecenoyl)-sn-glycerol, 1-hexadecanoyl-2-(9Z-octadecenoyl)-sn-glycero-3-phosphocholine, N-(tetradecanoyl)-sphing-4-enine, cholest-5-en-3β-ol, 2E,6E-farnesol, UDP-3-O-(3R-hydroxy-tetradecanoyl)-αd-N-acetylglucosamine, aflatoxin B₁, glycerophosphocholines, glycerophosphonocholines, glycerophosphoethanolamines, glycerophosphonoethanolamines, glycerophosphoserines, glycerophosphoglycerols, glycerophosphoglycerophosphates, glycerophosphoinositols, glycerophosphoinositol monophosphates, GPInsP, glycerophosphoinositol bis-phosphates, glycerophosphoinositol tris-phosphates, glycerophosphates, glyceropyrophosphates, glycerophosphoglycerophosphoglycerols, CDP-glycerols, glycerophosphoglucose lipids, glycerophosphoinositolglycans, ceramides, phosphosphingolipids, glycosphingolipids, monoradyl glycerols, diradyl glycerols, triradyl glycerols, fatty acids and conjugates, straight-chain fatty acids, methyl branched fatty acids, unsaturated fatty acids, hydroperoxy fatty acids, hydroxy fatty acids, oxo fatty acids, epoxy fatty acids, methoxy fatty acids, halogenated fatty acids, amino fatty acids, cyano fatty acids, nitro fatty acids, thia fatty acids, carbocyclic fatty acids, heterocyclic fatty acids, mycolic acids, dicarboxylic acids, octadecanoids, 12-Oxophytodienoic acid metabolites, Jasmonic acids, Eicosanoids, Prostaglandins, Leukotrienes, thromboxanes, lipoxins, hydroxyeicosatrienoic acids, hydroxyeicosatetraenoic acids, hydroxyeicosapentaenoic acids, epoxyeicosatrienoic acids, hepoxilins levuglandins, isoprostanes, clavulones, docosanoids, fatty alcohols, fatty aldehydes, fatty esters, wax monoesters, wax diesters, cyano esters, lactones, fatty acyl-CoAs, fatty acyl-acyl carrier proteins (ACPs), fatty acyl carnitines, fatty acyl adenylates, fatty amides, primary amides, N-acyl amides, fatty acyl homoserine lactones, N-acyl ethanolamides (endocannabinoids), fatty nitriles, fatty ethers, monoradylglycerols, monoacylglycerols, monoalkylglycerols, mono-(1Z-alkenyl)-glycerols, monoacylglycerolglycosides, monoalkylglycerolglycosides, diradylglycerols, diacylglycerols, alkylacylglycerols, dialkylglycerols, 1Z-alkenylacylglycerols, diacylglycerolglycosides, alkylacylglycerolglycosides, dialkylglycerolglycosides, di-glycerol tetraethers, di-glycerol tetraether glycans, triradylglycerols, triacylglycerols, alkyldiacylglycerols, dialkylmonoacylglycerols, 1Z-alkenyldiacylglycerols, estolides, glycerophosphocholines, diacylglycerophosphocholines, 1-alkyl,2-acylglycerophosphocholines, 1Z-alkenyl,2-acylglycerophosphocholines, dialkylglycerophosphocholines, monoacylglycerophosphocholines, 1-alkyl glycerophosphocholines, 1Z-alkenylglycerophosphocholines, glycerophosphoethanolamines, diacylglycerophosphoethanolamines, 1-alkyl,2-acylglycerophosphoethanolamines, 1Z-alkenyl,2-acylglycerophosphoethanolamines, dialkylglycerophosphoethanolamines, monoacylglycerophosphoethanolamines, 1-alkyl glycerophosphoethanolamines, 1Z-alkenylglycerophosphoethanolamines, glycerophosphoserines, diacylglycerophosphoserines, 1-alkyl,2-acylglycerophosphoserines, 1Z-alkenyl,2-acylglycerophosphoserines, dialkylglycerophosphoserines, monoacylglycerophosphoserines, 1-alkyl glycerophosphoserines, 1Z-alkenylglycerophosphoserines, glycerophosphoglycerols, diacylglycerophosphoglycerols, 1-alkyl,2-acylglycerophosphoglycerols, 1Z-alkenyl,2-acylglycerophosphoglycerols, dialkylglycerophosphoglycerols, monoacylglycerophosphoglycerols, 1-alkyl glycerophosphoglycerols, 1Z-alkenylglycerophosphoglycerols diacylglycerophosphodiradylglycerols, diacylglycerophosphomonoradylglycerols, monoacylglycerophosphomonoradylglycerols, glycerophosphoglycerophosphates, diacylglycerophosphoglycerophosphates, 1-alkyl,2-acylglycerophosphoglycerophosphates, 1Z-alkenyl,2-acylglycerophosphoglycerophosphates, dialkylglycerophosphoglycerophosphates, monoacylglycerophosphoglycerophosphates, 1-alkyl glycerophosphoglycerophosphates, 1Z-alkenylglycerophosphoglycerophosphates, glycerophosphoinositols, diacylglycerophosphoinositols, 1-alkyl,2-acylglycerophosphoinositols, 1Z-alkenyl,2-acylglycerophosphoinositols, dialkylglycerophosphoinositols, monoacylglycerophosphoinositols, 1-alkyl glycerophosphoinositols, 1Z-alkenylglycerophosphoinositols, glycerophosphoinositol monophosphates, diacylglycerophosphoinositol monophosphates, 1-alkyl,2-acylglycerophosphoinositol monophosphates, 1Z-alkenyl,2-acylglycerophosphoinositol monophosphates, dialkylglycerophosphoinositol monophosphates, monoacylglycerophosphoinositol monophosphates, 1-alkyl glycerophosphoinositol monophosphates, 1Z-alkenylglycerophosphoinositol monophosphates, glycerophosphoinositol bisphosphates, diacylglycerophosphoinositol bisphosphates, 1-alkyl,2-acylglycerophosphoinositol bisphosphates, 1Z-alkenyl,2-acylglycerophosphoinositol bisphosphates, monoacylglycerophosphoinositol bisphosphates, 1-alkyl glycerophosphoinositol bisphosphates, 1Z-alkenylglycerophosphoinositol bisphosphates, glycerophosphoinositol trisphosphates, diacylglycerophosphoinositol trisphosphates, 1-alkyl,2-acylglycerophosphoinositol trisphosphates, 1Z-alkenyl,2-acylglycerophosphoinositol trisphosphates, monoacylglycerophosphoinositol trisphosphates, 1-alkyl glycerophosphoinositol trisphosphates, 1Z-alkenylglycerophosphoinositol trisphosphates, glycerophosphates, diacylglycerophosphates, 1-alkyl,2-acylglycerophosphates, 1Z-alkenyl,2-acylglycerophosphates, dialkylglycerophosphates, monoacylglycerophosphates, 1-alkyl glycerophosphates, 1Z-alkenylglycerophosphates, glyceropyrophosphates, diacylglyceropyrophosphates, monoacylglyceropyrophosphates, glycerophosphoglycerophosphoglycerols (cardiolipins), diacylglycerophosphoglycerophosphodiradylglycerols, diacylglycerophosphoglycerophosphomonoradylglycerols, 1-alkyl,2-acylglycerophosphoglycerophosphodiradylglycerols, 1-alkyl,2-acylglycerophosphoglycerophosphomonoradylglycerols, 1Z-alkenyl,2-acylglycerophosphoglycerophosphodiradylglycerols, 1Z-alkenyl,2-acylglycerophosphoglycerophosphomonoradylglycerols, monoacylglycerophosphoglycerophosphomonoradylglycerols, 1-alkyl glycerophosphoglycerophosphodiradylglycerols, 1-alkyl glycerophosphoglycerophosphomonoradylglycerols, 1Z-alkenylglycerophosphoglycerophosphodiradylglycerols, 1Z-alkenylglycerophosphoglycerophosphomonoradylglycerols, CDP-glycerols, CDP-diacylglycerols, CDP-1-alkyl,2-acylglycerols, CDP-1Z-alkenyl,2-acylglycerols, CDP-dialkylglycerols, CDP-monoacylglycerols, CDP-1-alkyl glycerols, CDP-1Z-alkenylglycerols, glycerophosphoglucose lipids, diacylglycerophosphoglucose lipids, 1-alkyl,2-acylglycerophosphoglucose lipids, 1Z-alkenyl,2-acylglycerophosphoglucose lipids, monoacylglycerophosphoglucose lipids, 1-alkyl glycerophosphoglucose lipids, 1Z-alkenylglycerophosphoglucose lipids, glycerophosphoinositolglycans, diacylglycerophosphoinositolglycans, 1-alkyl,2-acylglycerophosphoinositolglycans, 1Z-alkenyl,2-acylglycerophosphoinositolglycans, monoacylglycerophosphoinositolglycans, 1-alkyl glycerophosphoinositolglycans, 1Z-alkenylglycerophosphoinositolglycans, glycerophosphonocholines, diacylglycerophosphonocholines, 1-alkyl,2-acylglycerophosphonocholines, 1 Z-alkenyl,2-acylglycerophosphonocholines, dialkylglycerophosphonocholines, monoacylglycerophosphonocholines, 1-alkyl glycerophosphonocholines, 1Z-alkenylglycerophosphonocholines, glycerophosphonoethanolamines, diacylglycerophosphonoethanolamines, 1-alkyl,2-acylglycerophosphonoethanolamines, 1Z-alkenyl,2-acylglycerophosphonoethanolamines, dialkylglycerophosphonoethanolamines, monoacylglycerophosphonoethanolamines, 1-alkyl glycerophosphonoethanolamines, 1Z-alkenylglycerophosphonoethanolamines, di-glycerol tetraether phospholipids (caldarchaeols), glycerol-nonitol tetraether phospholipids, and oxidized glycerophospholipids, sphingoid bases, sphing-4-enines (sphingosines), sphinganines, 4-hydroxysphinganines (phytosphingosines), sphingoid base homologs and variants, sphingoid base 1-phosphates, lysosphingomyelins and lysoglycosphingolipids, N-methylated sphingoid bases, sphingoid base analogs, ceramides, N-acylsphingosines (ceramides), N-acylsphinganines (dihydroceramides), N-acyl-4-hydroxysphinganines (phytoceramides), acylceramides, ceramide 1-phosphates, phosphosphingolipids, ceramide phosphocholines (sphingomyelins), ceramide phosphoethanolamines, ceramide phosphoinositols, phosphonosphingolipids, neutral glycosphingolipids, simple Glc series (GlcCer, LacCer, etc.), GalNAc_1-3Gal_1-4Gal_1-4Glc- (globo series), GalNAc_1-4Gal_1-4Glc- (ganglio series), Gal_1-3GlcNAc_1-3Gal_1-4Glc- (lacto series), Gal_1_4GlcNAc_1-3Gal_1-4Glc- (neolacto series), GalNAc_1-3Gal_1-3Gal_1-4Glc- (isoglobo series), GlcNAc_1-2Man_1-3Man_1-4Glc- (mollu series), GalNAc_1-4GlcNAc_1-3Man_1-4Glc- (arthro series), Gal- (gala series), acidic glycosphingolipids, gangliosides, sulfoglycosphingolipids (sulfatides), glucuronosphingolipids, phosphoglycosphingolipids, basic glycosphingolipids, amphoteric glycosphingolipids, arsenosphingolipids, sterols, cholesterol and derivatives, cholesteryl esters, phytosterols and derivatives, marine sterols and derivatives, fungal sterols and derivatives, steroids, C18 steroids (estrogens) and derivatives, C19 steroids (androgens) and derivatives, C21 steroids (gluco/mineralocorticoids, progestogins) and derivatives, secosteroids, Vitamin D2 and derivatives, Vitamin D3 and derivatives, bile acids and derivatives, C24 bile acids, alcohols, and derivatives, C26 bile acids, alcohols, and derivatives, C27 bile acids, alcohols, and derivatives, C28 bile acids, alcohols, and derivatives, Steroid conjugates, Glucuronides, Sulfates, Glycine conjugates, taurine conjugates, hopanoids, isoprenoids, C5 isoprenoids, C10 isoprenoids (monoterpenes), C15 isoprenoids (sesquiterpenes), C20 isoprenoids (diterpenes), C25 isoprenoids (sesterterpenes), C30 isoprenoids (triterpenes), C40 isoprenoids (tetraterpenes), polyterpenes, Quinones and hydroquinones, ubiquinones, Vitamin E, Vitamin K, polyprenols, bactoprenols, bactoprenol monophosphates, bactoprenol diphosphates, phytoprenols, phytoprenol monophosphates, phytoprenol diphosphates, dolichols, dolichol monophosphates, dolichol diphosphates, saccharolipids, acylaminosugars, monoacylaminosugars, diacylaminosugars, triacylaminosugars, tetraacylaminosugars, pentaacylaminosugars, hexaacylaminosugars, heptaacylaminosugars, acylaminosugar glycans, acyltrehaloses, acyltrehalose glycans, polyketides, macrolide polyketides, aromatic polyketides, nonribosomal peptide/polyketide hybrids. Accordingly, the lipid derivative according to the present invention may be a derivative of any lipid disclosed in the present application; in particular the lipid may be an alkyne derivative of any lipid disclosed herein. In certain aspects the alkyne lipid derivative may be selected from the group consisting of the list of lipids disclosed in this paragraph.

In further embodiments of the method the azido coumarin derivative is a 3-azido hydroxy coumarin. In some embodiments of the method the azido coumarin derivative is a 4-azido hydroxy coumarin.

In certain embodiments, the coumarin derivative is selected from the group consisting of 3-, 4,-, 5-, 6-, 7-, and 8-hydroxy azido coumarin. In some embodiments, the azido coumarin is 3-azido 7-hydroxy coumarin. In certain embodiments, the azido coumarin is selected from the group of 3-azido-7-dialkylamino-coumarins. In further embodiments, the azido coumarin is selected from the group consisting of 3-azido coumarin, 4-azido coumarin, 8-bromo 3-azido coumarin, 3-azido 7-methoxy coumarin, 3-azido 6-ethoxy coumarin, 3-azido-7-diethylamino-coumarin, and 3-azido-7-acetoxy-coumarin.

K. Sivakumar et al. (2004) Org. Lett. 6 (24) 4603-4610 (see also the supporting information) and Majumdar KC and Mondal S (2009) Letters in Organic Chemistry, 6, 82-87 disclose suitable azido coumarin compounds and methods of their synthesis and analysis.

In various embodiments, reaction step a) comprises the addition of a Cu(I) salt or complex.

In certain embodiments, the reaction solvent comprises acetonitrile. In some embodiments, the reaction solvent comprises acetonitrile falling within the range of 5 to 50 v/v%, or 10 to 50 v/v%. In certain embodiments, the reaction solvent comprises 25 v/v% acetonitrile. Large amounts of acetonitrile stabilize Cu(I).

The term "catalytic amount" refers to any amount of Cu(I) that provides the desired increase in the rate of the "click" reaction, meaning the increase in the rate of the formation of the triazolyl coumarin compound derivative of the at least one lipid.

Typically, the Cu(I) compound is present in a concentration range of 0.1 - 2 mM.

In some embodiments, reaction step a) comprises the addition of Cu(I), wherein the Cu(I) is used in form of a salt or complex. Such a salt or complex may be selected from the group consisting of CuI, CuBr, CuCl, CuOTf*C₆H₆, [Cu(NCCH₃)₄], and Cu[acetonitrile]₄BF₄ and Cu[acetonitrile]₄PF₆. Also possible is the use of combinations or mixtures of Cu(I) salts or complexes, such as the afore-mentioned.

In certain embodiments, the base of step i) is selected from the group of volatile and non-fluorescent bases.

Generally, any volatile and non-fluorescent alkyl amine may be chosen as a base according to the present invention.

As used herein, the term "alkyl amine" in some aspects refers to a straight or branched, saturated or unsaturated, molecule having 1-10 or more carbon atoms and one or more amino groups. The alkyl portion of the alkyl amine can be methyl, ethyl, propyl, butyl, pentyl, hexyl, iso-propyl, iso-butyl, sec-butyl, tert-butyl, etc. The amino groups can be primary, secondary, or tertiary. The alkyl amine can be further substituted with one or more (e.g., 1, 2, 3, or more) substituents.

In various embodiments, the base is selected from the group of bases consisting of N,N-diisopropylethylamine, triethylamine, trimethylamine, monoethylamine (MEA), diethylamine (DEA), triethylamine (TEA), monoisopropylamine, monoisopropylamine (MIPA), diisopropylamine (DIPA), dimethylaminopropylamine (DEAPA), dimethyl dipropylene Triamine (DMAPAPA), 3-Isopropoxypropylamine (IPOPA), 3-methoxypropylamine (MOPA), ethylmethylamine (EMA), tetramethylpropylenediamine (TMPDA), sec-butylamine (B2A), aminopropyldiethanolamine (APDEA), dimethyl isopropyl amine (DMIPA), dimethylethylamine (DMEA), diethylhydroxylamine (DEHA), and diethylhydroxylamine (DEHA). Combinations of the afore-mentioned bases are also contemplated herein. The person skilled in the art will appreciate that other alkyl amines are also useful in the present invention.

In further embodiments, the lipid of the lipid derivative is selected from the group consisting of oleate, palmitate, cholesterol, cholesterol ester, cardiolipin, diacylglycerol, phosphatidylcholine, phosphatidylethanolamine, phosphatidylglycerol, phosphatidylinositol, phosphatidylserine, triacylglycerol, and sphinganin.

In some embodiments, the method comprises, prior to step a), extracting the at least one lipid derivatized with at least one terminal alkyne group from a cell.

The term "cell" includes any cell, including bacteria, yeast, fungi, plant, insect, and mammalian cells, amongst others. In a preferred embodiment, the cell is a mammalian cell, in particular a human cell. The cell may be part of a tissue or organ.

The cell may be selected from, but is not limited to, CHO cells, in particular CHO K-1 and CHO DG44, BHK cells, NSO cells, SP2/0 cells, HEK293 cells, HEK293EBNA cells, PER.C6 cells, COS cells, 3T3 cells, YB2 cells, HeLa cells, and Vero cells. In certain aspects, the cell is selected from DHFR-deficient CHO cells, such as dhfr-CHO and CHO K-1.

In other embodiments, the host cell is an amphibian cell. Preferably, the cell is selected from, but not limited to, Xenopus laevis oocytes.

In still other embodiments, the host cell is an insect cell. The cell may be selected from, but is not limited to, Sf9, Sf21, and Tn5.

In other embodiments, the host cell is a plant cell. The cell may be selected from, but is not limited to, cells derived from Nicotiana tabacum, the acquatic plant Lemna minor or the moss Physcomitrella patens. These cells are known as a system for producing polypeptides, and may be cultured also as calli.

In different embodiments, the host cell is a lower eukaryotic cell. Lower eukaryotic cells according to the invention include, but are not limited to, unicellular, multicellular, and filamentous fungi, preferably selected from: Pichia sp. Candida sp. Saccharomyces sp., Saccharomycodes sp., Saccharomycopsis sp., Schizosaccharomyces sp., Zygosaccharomyces sp. Yarrowia sp., Hansenula sp., Kluyveromyces sp., Trichoderma sp, Aspergillus sp., and Fusarium sp. and Myceteae, preferably selected from Ascomycetes, in particular Chysosporium lucknowense, and Basidiomycetes, in particular Coniphora sp. as well as Arxula sp.

In specific embodiments thereof the cell is selected from, but not limited to, P. pastoris, P. stiptis, P. methanolica, P. bovis, P. canadensis, P. fermentans, P. membranaefaciens, P. pseudopolymorpha, P. quercuum, P. robertsii, P. saitoi, P. silvestrisi, P. strasburgensis; P. finlandica, P. trehalophila, P. koclamae, P. opuntiae, P. thermotolerans, P. salictaria, P. guercuum, P. pijperi; C. albicans, C. amphixiae, C. atlantica, C. corydalis, C. dosseyi, C. fructus, C. glabrata, C. fermentati, C. krusei, C. lusitaniae, C. maltosa, C. membranifaciens, C. utilis; S. bayanus, S. cerevisiae, S. bisporus, S. delbrueckii, S. fermentati, S. fragilis, S. mellis, S. rosei; Saccharomycodes ludwigii, Saccharomycopsis capsularis; Schizosaccharomyces pombe, Schizosaccharomyces octosporus, Zygosaccharomyces bisporus, Zygosaccharomyces mellis, Zygosaccharomyces rouxii; Yarrowia fipolytica, Hansenula polymorpha, Kluyveromyces sp., Trichoderma reseei., A. nidulans, A. candidus, A. carneus, A. clavatus, A. fumigatus , A. niger, A. oryzae, A. versicolor, Fusarium gramineum, Fusarium venenatum, and Neurospora crassa as well as Arxula adeninivorans.

In certain embodiments, the method comprises, prior to step a), extracting the at least one lipid derivatized with at least one terminal alkyne group from a cell which has been incubated with at least one lipid derivatized with at least one terminal alkyne group. The incubation may be generally done by supplementing a growth or maintenance medium of the cells with the lipid derivative and incubating the cells for a period of time sufficient for the derivatized lipid to be metabolized by the cells.

In various embodiments, the method comprises prior to step a) extracting the at least one lipid derivatized with at least one terminal alkyne group from a cell which has been incubated with at least one lipid derivatized with at least one terminal alkyne selected from the group consisting of terminal alkyne cholesterol derivative, omega alkyne oleate, omega alkyne palmitate, omega alkyne sphinganin, omega alkyne fatty acid, and omega alkyne unsaturated fatty acid.

The term alkyne oleate and omega alkyne oleate refers to oleate and oleic acid having an alkyne group bound to the omega carbon atom.

The term alkyne palmitate and omega alkyne palmitate refers to palmitate and palmitic acid having an alkyne group bound to the omega carbon atom.

In another aspect, the present invention relates to alkyne-modified lipid compounds. Encompassed by the present invention is a compound having the structural formula: 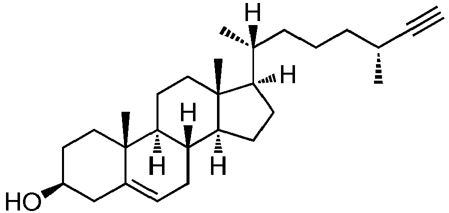 and the salts and/or esters thereof.

This compound is particularly advantageous over any other known cholesterol derivative, because it can be easily labeled, e.g., with a coumarin derivative, while structurally maintaining the cholesterol configuration as closely as possible. Especially, the correct C25 R configuration is maintained. So far, there is no other compound available which can be labeled and is structurally as much related to cholesterol. In particular, the compound biosynthetically mimics cholesterol and is therefore metabolized by cholesterol converting cells.

Another compound of the invention has the structural formula: 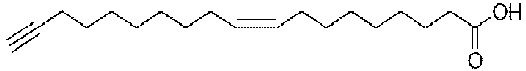 and the salts and/or esters thereof.

Still another compound has the structural formula: 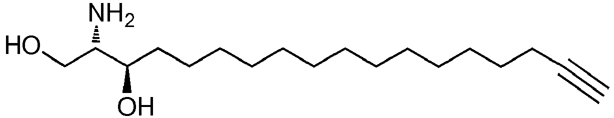 and the salts and/or esters thereof.

The term "salt" as used herein is not particularly restricted but involves any pharmacologically acceptable salts. Particular examples thereof include salts with inorganic bases containing metals such as sodium, potassium, magnesium, calcium and aluminum, salts with organic bases such as methylamine, ethylamine, ethanolamine, lysine and ornithine, and ammonium salts, salts with organic acids such as acetates, lactates, and citrates.

The term "ester", without being limited to, comprises formates, acetates, propionates, and butyrates of the compounds of the present invention.

Also encompassed by the present invention are the products of a reaction of the above compounds of the invention with azido modified coumarin compounds, such as 3,-, 4-, 5-, 6-, 7-, or 8-hydroxy azido coumarins. Alternatively, the products may be products of the alkyne lipid derivatives of the invention with azido modified fluorophores. The resulting product would be a fluorescently tagged lipid which is via a terminally coupled triazole group covalently bound to a fluorophore. Such fluorescently labeled lipid derivatives are also referred to herein as "fluorescent adducts".

The fluorophore may include, but is not limited to, fluorescein, rhodamine, Alexa Fluor, Dylight fluor, ATTO Dyes, IRIS dye, and/or Cy Dye, Cy2, Cy3, Cy5, Cy5.5, Cy7, Alexa405, Alexa430, Alexa488, Alexa500, Alexa514, Alexa532, Alexa546, Alexa568, Alexa594, Alexa610, Alexa633, Alexa647, Alexa660, Alexa680, Alexa700, and Alexa750, a quantum dot and combinations thereof. In some embodiments, the fluorophore is a coumarin derivative as disclosed herein.

In another aspect, the present invention relates to the use of a compound of the invention, i.e. the fluorescent adducts of the invention, in a fluorescence assay.

In a further aspect, the present invention relates to the use of a compound of the invention, e.g. the fluorescent adducts of the invention, for determining an enzyme activity, such as the catalytic activity of an enzyme. The enzyme may be involved in lipid metabolism.

The term "catalytic activity" as used herein refers to the increase in the rate of reaction of a specified chemical reaction that an enzyme produces in a specific assay system.

An assay system may comprise spectroscopic techniques, including fluorescence spectroscopy, e.g., FRET assays, calorimetry, surface plasmon resonance, and Enzyme-linked Immunosorbent Assays (ELISA).

In another aspect, the present invention relates to the use of a compound of the invention, e.g. the fluorescent adducts of the invention for analyzing or monitoring the lipid metabolism in a cell, tissue, organ or organism.

In a further aspect, the present invention relates to the use of a compound of the invention, i.e. the alkyne-modified lipids of the invention, for synthesis of derivatives thereof. The derivatives may be produced by cellular pathways of lipid metabolism and may, for example, include fatty acid containing cellular lipid products, such as phosphatidylcholines, phosphatidylethanolamines, phosphatidylserines, diacylglycerols, triacylglycerols, etc.

In another aspect, the present invention relates to a method of synthesis of alkyne lipids derivatives comprising contacting a cell with at least one compound selected from the group consisting of alkyne cholesterol, omega alkyne oleate, omega alkyne palmitate and alkyne sphinganin, cultivating the cell under conditions wherein the at least one compound is subjected to the cell's lipid metabolism, and extracting the alkyne derivatives produced by the cell. In some embodiments, the accordingly synthesized alkyne lipid derivatives are detected using the method disclosed herein.

In a further aspect, the present invention relates to a method of synthesis of a compound having a structural formula CH≡C-(CH₂)ₙ-COOH, wherein n is an integer selected from the group consisting of 1, 2, 3, 4, 5, 6, 7, 8, 9, 10, 11, 12, 13, 14, 15, 16, 17, 18, 19, 20, 21, and 22, comprising
d) reacting a compound having a formula Br-CH₂-CH₂-(CH₂)ₙ-COOH with KOH to give HO-CH₂-(CH₂)ₙ-COOH,
e) reacting the product of step a) with ROH, H⁺ to give HO-CH₂-(CH₂)ₙ-COO-R, wherein R is selected from the group comprising -CH₃, -CH₂CH₃, -n-propyl, -i-propyl, -n-butyl, -sec-butyl, and -t-butyl,
f) reacting the compound of step b) with pyridinium chlorochromate (PCC) in the presence of powdered molecular sieves to give O=CH-CH₂-(CH₂),-COO-R,
d) reacting the compound of step c) with potassium carbonate, dimethyl (1-diazo-2-oxopropyl)phosphonate (Bestmann-Ohira reagent), and methanol to give CH≡C-CH₂-(CH₂)ₙ-COO-R, and
e) subjecting the product of step d) to ester hydrolysis to give CH≡C-CH₂-(CH₂)ₙ-COOH.

In another aspect, the present invention relates to a method of synthesis of omega alkyne unsaturated fatty acids comprising
a) reacting a compound having a formula of HC≡C-(CH₂)ₙ-CH₂-OH with pyridinium chlorochromate (PCC) in DCM in the presence of powdered molecular sieves to give HC=C-(CH₂)ₙ-CH=O, wherein n is an integer selected from the group consisting of 1, 2, 3, 4, 5, 6, 7, 8, 9, 10, and 11,
b) reacting a compound having a formula of Br-CH₂-(CH₂)ₘ-CH₂-OH with CrO₃/H₂SO₄ to give Br-CH₂-(CH₂)ₘ-COOH, wherein m is an integer selected from the group consisting of 1, 2, 3, 4, 5, 6, 7, 8, 9, and 10,
c) reacting Br-CH₂-(CH₂)ₘ-COOH with triphenylphosphin at a temperature of 80-150°C to give the corresponding bromine salt of the omega triphenylphosphinyl fatty acid derivative, and
d) reacting the product of step c) with two equivalents of lithium hexamethylene-disilazane in dry THF under argon, cooling the reaction mix to -55°C, adding 1,3-dimethyl-3,4,5,6-tetrahydro-2(1H)-pyrimidinone (DMPU), followed by addition of the product of step a) solubilized in THF to give HC≡C-(CH₂)ₙ-CH=CH-(CH₂)ₘ-COOH.

In a further aspect, the present invention relates to a method of synthesis of omega alkyne unsaturated fatty acids comprising
a) reacting a compound having a formula of HO-CH₂-(CH₂)ₙ-CH₂-OH with propionic acid and toluenesulfonic acid to give CH₃C(=O)O-CH₂-(CH₂)ₙ-CH₂-OH, wherein n is an integer selected from the group consisting of 1, 2, 3, 4, 5, 6, 7, 8, 9, 10, 11, and 12,
b) reacting the compound of step a) with pyridinium chlorochromate (PCC) in DCM n the presence of powdered molecular sieves to give CH₃C(=O)O-CH₂-(CH₂)ₙ-CH=O,
c) reacting a compound having a formula of Br-CH₂-(CH₂)ₘ-CH₂-OH with CrO₃/H₂SO₄ to give Br-CH₂-(CH₂)ₘ-COOH, wherein m is an integer selected from the group consisting of 1, 2, 3, 4, 5, 6, 7, 8, 9, 10, 11, and 12,
d) reacting Br-CH₂-(CH₂)ₘ-COOH with triphenylphosphin at a temperature of 80-150°C to give the corresponding bromine salt of the omega triphenylphosphinyl fatty acid derivative,
e) reacting the product of step b) in dry THF under argon with two equivalents of lithium hexamethylene-disilazane, cooling the reaction mix to -55°C, adding 1,3-dimethyl-3,4,5,6-tetrahydro-2(1H)-pyrimidinone (DMPU), followed by addition of the product of step b) solubilized in THF to give CH₃C(=O)O-CH₂-(CH₂)ₙ-CH=CH-(CH₂)ₘ-COOH,
f) reacting the product of step e) with MeOH and acetylchloride to give *cis* HO-CH₂-(CH₂)ₙ-CH=CH-(CH₂)ₘ-COO-CH₃,
g) reacting the compound of step f) with pyridinium chlorochromate (PCC) in DCM in the presence of powdered molecular sieves to give cis O=CH-(CH₂)ₙ-CH=CH-(CH₂)ₘ-COO-CH₃,
h) reacting the compound of step g) with potassium carbonate, dimethyl (1-diazo-2-oxopropyl)phosphonate (Bestmann-Ohira reagent), and methanol to give *cis* CH≡C-(CH₂)ₙ-CH=CH-(CH₂)ₘ-COO-CH₃, and
i) reacting the product of step h) with KOH, MeOH, H₂O and HCl to give *cis* CH=C-(CH₂)ₙ-CH=CH-(CH₂)ₘ-COOH.

Step f) of the above method may further include purification of the product by chromatography on a AgNCV silica column before further use.

In another aspect, the present invention relates to a method of synthesis of (2S,3R)-2-aminooctadec-17-yn-1,3-diol (alkyne-sphinganine) comprising:
a) reacting THP-protected 1-tetradecyn-14-ol and Garners aldehyde to give *tert-Butyl* (4S,1'R)-4-(1'-hydroxypentadec-2'-yn-15-(2-tetrahydropyranyloxy)-yl-2,2-dimethyl-3-oxazolinecarboxylate,
b) reacting the product of step a) with H₂ in the presence of Pd/C to give tert-Butyl (4S,1'R)-4-(1'-Hydroxypentadec-15-(2-tetrahydropyranyloxy)-yl-2,2-dimethyl-3-oxazolinecarboxylate,
c) reacting the product of step b) with acetic anhydride in the presence of pyridine and DMAP to give tert-Butyl (4S,1'R)-4-(1'-Acetoxypentadec-15-(2-tetrahydropyranyloxy))-yl-2,2-dimethyl-3-oxazolinecarboxylate,
d) reacting the product of step c) with acetone, MeOH, and toluenesulfonic acid to give *tert-Butyl* (4S,1'R)-4-(1'-Acetoxypentadec-15-hydroxy)-yl-2,2-dimethyl-3-oxazolinecarboxylate,
e) reacting the product of step d) with pyridiniumchlorochromate (PCC) in the presence of powdered molecular sieves to give the corresponding aldehyde,
f) reacting the product of step e) with dimethyl (1-diazo-2-oxopropyl)phosphonate (Bestmann-Ohira reagent), potassium carbonate, and MeOH to give *tert-Butyl* (4S,1'R)-4-(1'-Hydroxyhexadec-15-yn)-yl-2,2-dimethyl-3-oxazolinecarboxylate, and
g) reacting the product of step f) in THF with HCl to give (2S,3R)-2-Aminooctadec-17-yn-1,3-diol.

In a further aspect, the present invention relates to a method of synthesis of (25R)-25-ethinyl-26-nor-3b-hydroxycholest-5-en (click-Cholesterol) comprising:
a) reacting (25R)-3beta-(tert-Butyldimethylsilyloxy)cholest-5-en-26-ol with pyridiniumchlorochromate (PCC) in the presence of powdered molecular sieves to give (25R)-3b-(tert-Butyldimethylsilyloxy)cholest-5-en-26-al, and
b) reacting the product of step a) with dimethyl-(1-diazo-2-oxopropyl)phosphonate (Bestmann-Ohira reagent) potassium carbonate, MeOH, and THF, followed by the treatment of the resulting product with HCl and THF to give(25R)-25-ethinyl-26-nor-3b-hydroxycholest-5-en.

The reaction steps disclosed herein involving the use of triphenylphosphin may be performed in acetonitrile or toluene as a solvent. Preferably, the reaction is performed in acetonitrile.

The reaction steps disclosed herein involving the use of pyridinium chlorochromate (PCC) in the presence of powdered molecular sieves may be performed in DCM or acetonitrile as a solvent, both optionally supplemented with liquid alkanes, e.g. hexane. Preferably, the reaction is performed in DCM.

The skilled person is aware of suitable methods of ester hydrolysis. For example, the skilled person may hydrolyze an ester under basic conditions using KOH or NaOH.

As an alternative to TLC disclosed herein, in some embodiments HPLC may be applied. For example, HPLC separation with online fluorescence detection offers excellent resolution and sensitivity.

The method of the present invention opens numerous perspectives of further experiments. The compounds of the present invention allow for a highly specific and rapid analysis of lipid metabolism because a) the compounds mimic the endogenous lipids and are thus metabolized as their native counterparts and are b) detectable even in very low amounts using the methods disclosed herein. Evidently, its application is not confined to the feeding of fatty acids to living cells, but also includes study of the metabolism of labeled sphingolipids and sterols, as well as the use of alkyne fatty acyl-CoA to monitor acyl-transferase reactions in vitro. Also, instead of being used as standards, alkyne-fatty acid labeled compounds (Figure 7) can also serve as substrates to study lipases, phospholipases and other lipid-metabolizing enzymes. Finally, combination with light microscopic imaging is a genuine possibility for alkyne-lipids, not available for radiolabeled compounds. Furthermore, the alkyne compounds disclosed herein, e.g., alkyne-fatty acids, -cholesterol,-sphinganine, -oleate, and-palmitate, can be imaged after incorporation into cellular lipids by click-reaction with various fluorescent dyes, e.g. coumarin. The combination of the two methods has a large potential for studies in which spatial organization of lipid metabolism play a key role.

### EXAMPLES

### Materials & Methods

### Synthesis

### Cis-unsaturated terminal alkyne fatty acid

### (1) 9-Decynal

To a stirred mixture of 15 g powdered molecular sieves and 10 g (47 mmol) pyridinium chlorochromate in 100 ml DCM, 5 g (32 mmol) 9-decyn-1-ol were added dropwise under stirring at 20°C, and stirring was continued for 30 min. Completion of the reaction was controlled by TLC in hexane/ethyl acetate 3/1. The solvent was removed *in vacuo* and the residue extracted with 3 x 60 ml hexane/ethyl acetate 4/1. The extract was filtered through a 8 cm silica bed and evaporated to give 3.9 g pure **(1),** which was directly used for the following Wittig reaction.

### (2) Carboxyoctyl-triphenylphosphoniumbromide

A mixture of 2.47 g, (10 mmol) 9-Bromononanoic acid (obtained by oxidation of commercial 9-bromononanol with CrO₃/H₂SO₄ in acetone) and 2.62 g (10 mmol) triphenylphosphin in 10 ml acetonitrile was heated in a closed teflon reaction chamber to 120°C for 16 h. The solvent was removed *in vacuo* and the residue slowly mixed with ether and ethyl acetate until a precipitate formed. Residual solvent was removed *in vacuo* to give 4.9 g of (2) as a foamy semisolid. 1H-NMR (400 MHz, CDCl₃) 7.8-7.6 ppm (m, 15H, phenyl-H), 3.55 (m, 2H, C9H₂), 2.25 (t, 2H, C2H₂), 1.5-1.2 (m,b 12H, C3-8H₂)

### (3) Nonadec-9-cis-en-18-ynoic acid (Alkyne-Oleate, aOle)

To a suspension of 9.0 g (18 mmol) of (**2**) in 200 ml dry THF under argon, a solution of lithium hexamethylene-disilazane (1 M, about 38 ml) was added until the entire amount of (**2**) was dissolved to form an orange-red solution, which required extended sonication in a water bath at room temperature. After cooling to -55°C, 125 ml of 1,3-Dimethyl-3,4,5,6-tetrahydro-2(1H)-pyrimidinone (DMPU) were added, followed by addition of a solution of 3.8 g (25 mmol) (**1**) in 10 ml THF, all under inert gas. The mixture was stirred for 30 min and was allowed to come to room temperature. THF was removed *in vacuo* and the residue acidified with 100 ml 1 M HCl and 100 ml water. The resulting turbid suspension was extracted with 2 x 150 ml hexane/ethyl acetate 3/1 and the extract was washed with water and the solvent removed *in vacuo.* The residue was purified by silica column chromatography (solvent hexane/ethyl acetate 3/1). Residual impurities were removed by the following procedure: 4 g of the raw product were dissolved in 100 ml methanol and 40 ml water and brought to pH 10 by addition of NaOH. This solution was washed with 3x100 ml hexane/ethyl acetate 3/1. The methanol-water phase was re-acidified with 1 M HCl to pH 2 and extracted with 2 x 150 ml hexane/ethyl acetate 3/1. The combined extracts were washed with water and evaporated to give 3.6 g (12.3 mmol) product. 1H-NMR (400 MHz, CDCl₃) 5.33 (m, 2H, trans/cis C9,10*H*, ratio trans/cis 5/95), 2.33 (t, 2H, C2*H*₂), 2.16 ppm (dt, 2H, C17*H*₂), 2.05-1.95 (m,b 4H, C8,11*H*₂), 1.91 (t, 1H, C19*H*), 1.62 (m, 2H, C3*H*₂)-1.50 (m, 2H, C16*H*₂), 1.4-1.2 (m,b 16H, C4-7,12-15*H*₂). 13C-NMR (400 MHz, CDCl₃): 180.19 ppm (C1), 129.87 (C10), 129.79 (C9), 84.63 (C18), 68.04 (C19), 34.04 (C2), 29.66 (C7 + C12), 29.12 (C5 + C14), 29.05 + 29.01 + 28.98 (C4 + C6 + C13), 28.71 (C16), 28.46 (C15), 27.15 + 27.14 (C8 + C11), 24.64 (C3), 18.37 (C17).
Pos. ion LC-MS: expected m/z = 310.2746 [M + NH4]⁺.

### Saturated terminal alkyne fatty acids

Saturated alkyne fatty acids were synthesized starting from the omega-hydroxy fatty acids by methyl ester formation, PCC oxidation, Bestmann-Ohira reaction and ester cleavage.

### (4) 8-Nonynoic acid

a) 8-Hydroxyoctanoic acid. Commercial 8-bromooctanoic acid (4.5 g, 20 mmol) was mixed with 3 g KOH in 70 ml water and 30 ml THF and stirred at 80°C for 4h. After acidification with HCl, the mixture was extracted with hexane/ethyl acetate 1/1, the solvent was removed *in vacuo* and the residue purified by silica column chromatography (solvent hexane/ethyl acetate 1/1).
b) Methyl-8-hydroxyoctanoate. The hydroxy acid was dissolved in 80 ml MeOH plus 1 ml acetyl chloride, stirred for 1h and evaporated to obtain the methyl ester.
c) 1-Carboxymethyl-7-heptanal. The methyl ester was dissolved in 40 ml dichloromethane, and 3 g powdered molecular sieves 4Å and 3.5 g pyridinium chlorochromate were added. After stirring for 1h at room temperature, the solvent was evaporated and the residue extracted with 3 x 50 ml hexane/ethyl acetate 3/1. The combined extracts were filtered through a 4 cm silica bed and evaporated to give 1.6 g of 1-carboxymethyl-7-heptanal.
d) Methyl-8-nonynoate. The aldehyde was immediately dissolved in 100 ml methanol and stirred with 2.2 g potassium carbonate and 2 g Dimethyl (1-Diazo-2-oxopropyl)phosphonate (Bestmann-Ohira reagent) for 16 h. After addition of 100 ml water, the mixture was extracted with 3 x 100 ml hexane/ethyl acetate 2/1, which was evaporated to give 1.4 g of a residue (methyl-8-nonynoate), which was dissolved in 30 ml MeOH.
e) 8-Nonynoic acid. After addition of 30 ml water and 2 g KOH and stirring for 3 h at 45°C, the mixture was acidified with HCl. After addition of 50 ml brine, the mixture was extracted with 3 x 100 ml hexane/ethyl acetate 2/1, the organic phases were evaporated and the residue purified by silica column chromatography (solvent hexane/ethyl acetate/AcOH 200/100/1) to give 1.1 g pure 8-nonynoic acid.
1H-NMR (400 MHz, CDCl₃) 2.34 (t, 2H, C2*H*₂), 2.16 ppm (dt, J=7.2 Hz, 2.7 Hz, 2H, C7*H*₂), 1.92 (t, J=2.7 Hz, 1H, C9*H*), 1.63 (m, 2H, C3*H*₂),1.52 (m, 2H, C6*H*₂), 1.45-1.2 (m, 4H, C4,5*H*₂).

### (5) 12-Tridecynoic acid

Synthesis according to (**4**) from 2.7 g of commercial 12-hydroxydodecanoic by acid methyl ester formation, pyridinium chlorochromate oxidation, Bestmann-Ohira reaction and ester cleavage to give 1.2 g product.
1H-NMR (400 MHz, CDCl₃) 2.30 (t, 2H, C2H₂), 2.14 ppm (dt, J=7.1 Hz, 2.6 Hz, 2H, C11H₂), 1.91 (t, J=2.6 Hz, 1H, C13H), 1.59 (m, 2H, C3H₂), 1.48 (m, 2H, C10H₂), 1.4-1.2 (m, 12H, C4-9H₂).

### (6) 16-Heptadecynoic acid (Alkyne-Palmitate, aPal)

Synthesis according to **(4)** from commercially available methyl-juniperate by pyridinium chlorochromate oxidation, Bestmann-Ohira reaction and ester cleavage.
1H-NMR (400 MHz, CDCl₃) 2.32 (t, 2H, C2*H*₂), 2.15 ppm (dt, J=5.5 Hz, 2.6 Hz, 2H, C15*H*₂)_{,} 1.91 (t, J=2.6 Hz, 1H, C17*H*), 1.61 (m, 2H, C3*H*₂), 1.50 (m, 2H, C14*H*₂), 1.4-1.2 (m,b 20H, C4-13*H*₂)_{.}
Pos. ion LC-MS: expected m/z = 284.2584 [M + NH₄]⁺.

### Synthesis of alkyne-oleate labeled TLC standards

**TAG:** 15 mg (0.05 mmol) alkyne-oleate were reacted with 11.3 mg (0.07 mmol) carbonyldiimidazol in 50 µl THF for 10 min at 30°C. A solution of 60 mg (0.1 mmol) dioleoyl-diacylglycerol (mixed 1,2 and 1,3 isomers) in 50 µl THF was added and the mixture incubated at room temperature for 2h. Labeled TAG was purified by silica column chromatography (solvent hexane/ethyl acetate 6/1) to yield 28 mg product. The identity was confirmed by co-migration with authentic TAG on TLC plates and can be confirmed by mass spectrometry.
Pos. ion LC-MS: expected m/z = 912.8020 [M + NH₄]⁺.

**DAG:** As described above for TAG, but 1-oleoylglycerol was used as acylation substrate.
Pos. ion LC-MS: expected m/z = 648.5567 [M + NH₄]⁺.

**1-MAG:** As described above for TAG, but sn-2,3-isopropylideneglycerol was used as acylation substrate, followed by deprotection with HCl in THF/H₂O.
Pos. ion LC-MS: expected m/z = 384.3114 [M + NH₄]⁺.

**CE** (Alkyne-oleoyl-cholesterol): As described above for TAG, but cholesterol was used as acylation substrate.
Pos. ion LC-MS: expected m/z = 678.6189 [M + NH₄]⁺.

**PA:** 10 mg 1-oleoyl-2-(alkyne-oleoyl)glycerol (containing about 10 % 1,3-isomer) were dissolved in 100 µl DCM and 10 µl pyridine. This solution was added to a mixture of 5 µl POCl₃ in 100 µl DCM. After 30 min, 10 µl H₂O were added and the mixture vigourously shaken for 10 min. PA was isolated by silica gel chromatography using CHCl₃/MeOH/H₂O 65/25/4 as a solvent.
Neg. ion LC-MS: expected m/z = 709.4818 [M]⁻.

**PC:** Lyso-PC (mixed fatty acids on sn-1, from egg yolk) was acylated with alkyne-oleate, DCC and DMAP as described in Menger and Wong, J. Org. Chem. 1996, 61, 7382-7390 and purified by silica gel chromatography using CHCl₃/MeOH/H₂O 65/25/4 as a solvent.
Pos. ion LC-MS: expected major peak m/z = 770.5694 [PC 16:0,19:3 + H]⁺.

**Alkyne-Bodipy** (8-(4-pentinyl)-4,4-difluor-1,3,5,7-tetramethyl-4-bora-3a,4a-s-indacen):

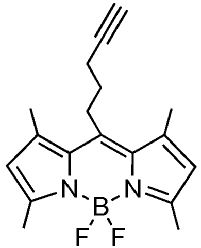

The substance was synthesized from 5-hexynoyl chloride, tetramethylpyrrole, BF₃xEt₂O and triethylamine according to the general procedure given in Spandl J et al, Traffic 2009, 10, 1579-1584 and Morgan LR, Boyer JH, USA Patent 5,446,157, 1995.
1H-NMR (CDCl₃) 5.99 (s, 2H, C2,6*H*, 3.04 (m, 2H, C1'*H*₂), 2.44 (s, 6H, C10,11*H*₃), 2.37 (s, 6H, C9,12*H*₃), 2.33 (dt, 2H, C3'*H*₂), 1.98 (t, 1H, C5'*H*), 1.77 (m, 2H, C2'*H*₂).

### Synthesis of Click-Cholesterol

### (25R)-3b-(tert-Butyldimethylsilyloxy)cholest-5-en-26-al

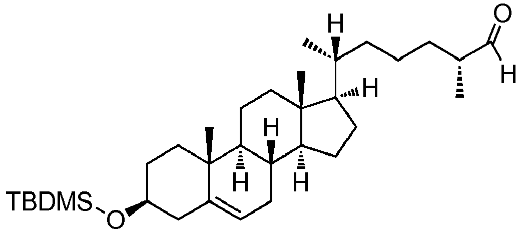

A mixture of 250 mg (0.48 mmol) (25R)-3b-(tert-Butyldimethylsilyloxy)cholest-5-en-26-ol (synthesized as described in Martin R et al., 2009. Org Biomol Chem 7, 909-920, compound 14 of this reference), 600 mg (2.78 mmol) pyridiniumchlorochromate, 3 g powdered molecular sieves and 8 ml dichloromethane was stirred at room temperature for 30 min. The solvent was evaporated, the residue extracted with 8 ml hexane/ethyl acetate 6/1 and purified by silica gel column chromatography using hexane/ethyl acetate 6/1 as a solvent to yield 180 mg product.
1H-NMR (400 MHz, CDCl₃): 9.56 ppm (d, 1H, 26-C*H*O), 5.26 (m, 1H, 6-C*H*), 3.42 (m, 1H, 3-C*H*), 1.04 (d, 3H. 27-C*H*₃), 0.94 (s, 3H, 19-C*H*₃), 0.85 (d, 3H, 21-C*H*₃), 0.82 (s, 9H, Si-C(C*H*₃)₃), 0.61 (s, 3H, 18-C*H*₃), 0.0 (s, 6H, Si-(C*H*₃)₂)

### (25R)-25-ethinyl-26-nor-3b-hydroxycholest-5-en

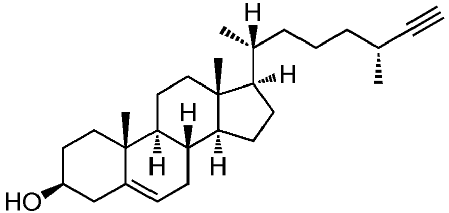

A mixture of 180 mg (0.35 mg) (25*R*)-3b-(*tert*-Butyldimethylsilyloxy)cholest-5-en-26-al, 110 mg (0.58 mmol) Dimethyl-(I-Diazo-2-oxopropyl)phosphonate (Bestmann-Ohira reagent, TCI), 80 mg (0.6 mmol) potassium carbonate and 6 ml MeOH/THF 1/1 was stirred for 5 h at 45°C. The solvent was evaporated, the remnant dissolved in hexane/ethyl acetate 5/1 1 and purified by silica gel column chromatography using hexane/ethyl acetate 6/1 as a solvent to yield 170 mg protected alkyne of 90% purity. This material was dissolved in 10 ml THF and 2 ml of 4N HCl were added. After 10 min, the deprotection reaction was stopped by addition of an excess of solid sodium carbonate. Ether (50 ml) was added and the mixture washed twice with 10 ml of brine. The solvent was evaporated and the remnant purified by silica gel column chromatography using hexane/ethyl acetate 3/1 as a solvent to yield 120 mg of final product.
1H-NMR (400 MHz, CDCl₃): 5.34 ppm (m, 1H, 6-C*H*), 3.50 (m, 1H, 3-C*H*), 2.40 (m, 1H, 25-CH), 2.015 (d, J=2.6 Hz, 1H, Ethinyl-C*H*), 1.14 (d, 3H. 27-C*H*₃), 0.99 (s, 3H, 19-C*H*₃), 0.91 (d, 3H, 21-C*H*₃), 0.66 (s, 3H, 18-C*H*₃),

### Synthesis of (2S,3R)-2-aminooctadec-17-yn-1,3-diol (alkyne-sphinganine)

*tert-Butyl* (4*S*,1'*R*)-4-(1'-Hydroxypentadec-2'-yn-15-(2-tetrahydropyranyloxy)-yl-2,2-dimethyl-3-oxazolinecarboxylate **(7)** was synthesized from THP-protected 1-tetradecyn-14-ol and Garners aldehyde according to Kozikowski et al, Tetrahedron Letters, Vol. 37, No. 19, pp. 3279-3282, 1996.

*tert-*Butyl (4*S*,1'*R*)-4-(1'-Hydroxypentadec-15-(2-tetrahydropyranyloxy)-yl-2,2-dimethyl-3-oxazolinecarboxylate **(8)**

400 mg of **(7)** were dissolved in 10 ml EtOH. After addition of 40 mg 10% Pd/C, the mixture was stirred for 2h at 1 bar H₂ at room temperature. 40 ml EtOH were added and the catalyst pelleted by centrifugation at 5000 g for 15 min. Evaporation of the solvent yielded the product in quantitative yield.

1H-NMR (400 MHz, CDCl₃) 4.55 (m, 1H, THP C2*H*), 4.1-3.3 (5 x m, 8H, C4*H*, C5*H*₂, C1'*H*, C15'*H*2, THP C6*H*₂), 1.8 and 1.68 (2 x m, 2H, THP C3*H*₂), 1.6-1.36 ppm (m, b, 23H, *C2'H₂,* C14'H₂, THP C4*H*₂, THP C5*H*₂, 5 x methyl-C*H*₃) 1.36-1.1 (m,b 22H, C3'-13'*H*₂),

*tert*-Butyl (4*S*,1'*R*)-4-(1'-Acetoxypentadec-15-hydroxy)-yl-2,2-dimethyl-3-oxazolinecarboxylate (**10**)

370 mg of **(8)** were dissolved in 5 ml DCM. After addition of 0.5 ml pyridine, 250 µl acetic anhydride and 40 mg DMAP, the mixture was stirred for 30 min at RT. After addition of 20 ml hexane/ethyl acetate 1/1 and 20 ml brine, the organic phase was collected and dried to give the acetylated product in high purity and yield. The residue **(9)** was dissolved in a mixture of 6 ml acetone, 2 ml MeOH and 30 mg toluenesulfonic acid. After stirring at RT for 2h, 20 ml hexane/ethyl acetate 1/1 and 10 ml sat. aq. NaHCO3 were added. The organic layer was separated, evaporated and the residue purified by silica **gel** chromatography (solvent gradient hexane/ethyl acetate 3/1 to 1/1) to give 230 mg pure **(10).**
1H-NMR (400 MHz, CDCl₃) 5.33 (m, 1H, C1'*H*), 4.1-3.8 (3 x m, 3H, C4*H*, *C5H₂),* 3.61 (t, 2H, C15'*H*₂), 2.04 (s, 3H, acetyl C*H*₃), 1.6-1.38 ppm (m, b, 19H, *C2'H₂,* C14'H₂, 5 x methyl-CH₃) 1.38-1.1 (m,b 22H, *C3'-13'H₂)*

*tert*-Butyl (4*S*,1'*R*)-4-(1'-Hydroxyhexadec-15-yn)-yl-2,2-dimethyl-3-oxazolinecarboxylate (**12**)
a) PCC oxidation to the aldehyde: 200 mg of **(10)** were dissolved in 5 ml DCM. After addition of 200 mg powdered molecular sieves and 220 mg pyridiniumchlorochromate, the mixture was stirred for 30 min at RT. The solvent was evaporated and the residue extracted with 3 x 10 ml hexane/ethyl acetate 3/1. The exctracts were evaporated and the aldehyde **(11)** isolated by silica gel chromatography (solvent hexane/ethyl acetate 1/1).
b) Bestmann-Ohira reaction: the aldehyde was stirred with 140 mg Dimethyl (1-Diazo-2-oxopropyl)phosphonate (Bestmann-Ohira reagent) and 200 mg potassium carbonate in 6 ml MeOH for 30 min at RT. TLC control showed formation of the alkyne and a weak band of lower mobility. Upon prolonged stirring (3h at RT, 1h at 45°C) more than 90% of the material was found in the lower band. After addition 20 ml hexane/ethyl acetate 1/1 and 20 ml brine, the organic phase was collected and dried and the residue purified by silica gel chromatography (solvent hexane/ethyl acetate 3/1) to give 130 mg pure (**12**)
1H-NMR (400 MHz, CDCl₃): 4.1-3.4 (m, 4H, C1'*H*, C4H, C5*H*₂), 2.16 ppm (dt, J=7.2 Hz, 2.7 Hz, 2H, C14'*H*₂), 1.92 (t, J=2.7 Hz, 1H, C16*'H*), 1.6-1.37 ppm (m, b, 19H, *C2'H₂,* C13*'H*₂, 5 x methyl-C*H*₃) 1.37-1.1 (m,b 20H, C3'-12'*H*₂)

### (2S,3R)-2-Aminooctadec-17-yn-1,3-diol (13)

100 mg of (6) were dissolved in 4 ml THF and 1 ml conc. HCl and stirred for 2h at RT. The solvent was evaporated *in vacuo,* the residue dissolved in 10 ml water and the water phase extracted with 10 ml hexane/diethyl ether 4/1. The aqueous phase was brought to pH 10 by addition of NaOH and extracted with 4 x 5 ml of DCM. The pooled DCM phases were evaporated and the residue purified by silica gel chromatography (solvent CHCl₃/MeOH/aq. NH₃ 40/10/1) to give 47 mg pure **(13).**
1H-NMR (400 MHz, (CD₃OD): 3.72 ppm (dd, 1H, C1a*H*), 3.50 (m, J=7.2, 1H, C3*H*), 3.46 (dd, 1H, C1b*H*), 2.71 (ddd, 1H, C2*H*), 2.15 (m, 3H, C16*H*₂, C17*H*), 1.6-1.45 ppm (m, b, 4H, C4*H*₂, C15*H*₂) 1.45-1.2 (m,b 20H, C5-14*H*₂)
13C-NMR (400 MHz, CDCl₃+10% CD₃OD): 84.75 ppm (C17), 73.88 (C3), 67.97 (C18), 63.05 (C1), 55.65 (C2), C4-C16: 33.66, 29.60, 29.54 (large signal), 29.51, 29.41, 29.02, 28.67, 25.95, 18.28.

### Alternative synthesis of alkyne-fatty acids

### Nonan-1,9-diol-monopropionate (14)

A mixture of nonandiole (36 g, 0.2 mol), propionic acid (7.5 g, 0.1 mol) and toluenesulfonic acid (1g) was boiled in an open round bottom flask until no water was released any more. The solution was cooled to 0°C and stirred for 15 min. Crystallized material (mainly unreacted diol; can be recycled for the same purpose) was collected by filtration. The filtrate was washed with sat. NaHCO₃ and brine, dried over MgSO₄ and the solvent evaporated *in vacuo.* The residue (20 g) was dissolved in 100 ml hexane and loaded on a 8 x 25 cm silica column in hexane/ethyl acetate 9/1, followed by elution with 300 ml hexane/ethyl acetate 9/1, 400 ml hexane/ethyl acetate 4/1 and 400 ml hexane/ethyl acetate 2/1 to yield 14.5 g pure (1). 1H-NMR (CDCl₃) 4.04 ppm (t, 2H, C1*H*2₎, 3.60 (t, 2H, C9*H*₂) 2.29 (q, 2H, propionyl-C2*H*₂), 1.65-1.48 (m, 4H, C2,8*H*₂) 1.35-1.25 (b, 10H, C3-7*H*₂), 1.11 (t, 3H, propionyl-C3*H*₃)

### 1-Propionoxy-nonan-9-al (15)

To a solution of 4.5 g (21 mmol) (1) in 60 ml DCM were added 15 g powdered molecular sieves and 6 g (28 mmol) pyridinium chlorochromate. The mixture was stirred at 20°C for 40 min. The solvent was evaporated and the residue extracted with 4 x 80 ml hexane/ethyl acetate 4/1. The extract was filtered through a 8 x 8 cm silica bed and evaporated to give 3.8 g pure **(15).** 1H-NMR (CDCl₃) 9.70 ppm (t, 1H, C9*H*), 4.02 ppm (t, 2H, C1*H*2), 2.38 (t, 2H, C8*H*₂), 2.27 (q, 2H, propionyl-C2*H*₂), 1.65-1.52 (m, 4H, C2,7*H*₂) 1.35-1.25 (b, 8H, C3-6*H*₂), 1.09 (t, 3H, propionyl-C3*H*₂)

### Carboxyoctyl-triphenylphosphoniumbromide (16)

9-Bromononanoic acid (2.47 g, 10 mmol, obtained by CrO₃/H₂SO₄ oxidation of commercial 9-bromononanol) and 2.62 g (10 mmol) triphenylphosphin were dissolved in 10 ml acetonitril and heated in a closed teflon reaction chamber to 120°C for 16 h. The solvent was removed in vacuo and the residue slowly mixed with ether and ethyl acetate until a precipitate formed, which was collected and dried to give 4.9 g of **(16)** as a honeylike semisolid. 1H-NMR (CDCl₃) 7.8-7.6 ppm (m, 15H, phenyl-*H*), 3.55 (m, 2H, C9*H*₂), 2.25 (t, 2H, C2*H*₂), 1.5-1.2 (m,b 12H, C3-8*H*₂)

### 18-propionoxy-9-octadecenoic acid (17)

4.9 g (9.6 mmol) of **(16)** were stirred at room temperature with 180 ml dry THF. AIM solution of lithium hexamethylene-disilazane (about 22 ml) was added until the entire amount of **(16)** was dissolved to form an orange-red solution. After cooling to -15°C, a solution of 2.1 g (10 mmol) **(15)** in 10 ml THF was added through a syringe under inert gas. After stirring for 30 min, the mixture was concentrated to 60 ml. After addition of 80 ml hexane, the solution was extracted twice with 2 x 150 ml water. The aqueous extract was acidified with HCl and extracted with 2 x 150 ml ether. The ether extract was washed with water and brine, dried over Na₂SO₄ and evaporated. The residue was purified by silica column chromatography (solvent hexane/ethyl acetate 3/1) to give 1.55 g of **(17).** 1H-NMR (CDCl₃) 5.26 and 5.23 (2 x m, overlapping, 2H, trans/cis C9,10*H*, ratio trans/cis 22/78), 3.95 ppm (t, 2H, C18*H*₂), 2.23 (m, 4H, C2*H*₂ and propionyl-C2*H*₂), 1.9 (m,b, 4H, C8,11*H*₂) 1.55-1.45 (m,b, 4H, C3,17*H*₂)_{,} 1.2 (m,b, 18H, C4-7,12-16*H*₂), 1.04 (t, 3H, propionyl-C3*H*₃)

### Methyl-18-Hydroxy-9-cis-octadecenoate (18)

To a mixture of 50 ml MeOH and 1 ml acetylchloride was added 1.5 g (4.2 mmol) of **(17).** Progress of the parallel methanolysis and methyl ester formation was followed by TLC. After 4 h at room temperature, the solvent was evaporated and the product purified by silica gel chromatography (solvent hexane/ethyl acetate 5/1 to 2/1) to yield 1.0 g pure **(18).** By NMR analysis of the olefinic protons, this substance had a trans/cis ratio of 20/80. To obtain the desired pure cis-compound, the substance was dissolved in solvent hexane/ethyl acetate 4/1 and subjected to chromatography on a 2.5 x 25 cm AgNCV silica column (made by boiling 100 g of silica in a solution of 100 g AgNO₃ in 200 ml water, filtration and drying of the impregnated silica for 16 h at 120°C), which was eluted with 100 ml hexane/ethyl acetate 4/1, 200 ml hexane/ethyl acetate 3/1 and 300 ml hexane/ethyl acetate 3/2. Fractions were analyzed by TLC on silver-impregnated silica plates. Pure fractions were pooled, and mixed fractions re-Chromatographed to obtain 700 mg product. 1H-NMR (CDCl₃) 5.31 (m, 2H, trans/cis C9,10*H*₂, ratio trans/cis 3/97), 3.64 (s, 3H, methyl-C*H*₂), 3.61 ppm (t, 2H, C18H₂), 2.28 (t, 2H, C2*H*₂) 2.05-1.9 (m,b 4H, C8,11*H*₂), 1.65-1.45 (m,b 4H, C3,17*H*₂), 1.2 (m,b 18H, C4-7,12-16*H*₂)

### Nonadec-9-cis-en-18-inoic acid (Alkyne-Oleate, aOle) (21)

A mixture of 650 mg (2.1 mmol) of (**18**), 650 mg (3 mmol) pyridinium chlorochromate and 1 g powdered molecular sieves in 6 ml DCM was stirred for 1 h at room temperature. The solvent was evaporated and the residue extracted with 3 x 5 ml hexane/ethyl acetate 3/1. The combines extracts were filtered through silica, evaporated and the residue directly used for the next step. To this were added 15 ml MeOH/THF 1/1, 575 mg (3 mmol) Dimethyl (1-Diazo-2-oxopropyl)phosphonate (Bestmann-Ohira reagent, obtained from TCI) and 900 mg (5 mmol) K₂CO₃. The mixture was stirred for 4h at 30°C, diluted with 100 ml ethyl acetate and 50 ml water, and the organic phase was collected and washed with water. After evaporation of the solvent, the residue was dissolved in 20 ml methanol and 1 ml 10 M KOH in water and stirred at 45°C for 2 h. The mixture was acidified with HCl, diluted with 20 ml water and extracted with 3 x 30 ml ethyl acetate. The combined extracts were dried and the residue was purified by silica column chromatography (solvent hexane/ethyl acetate 3/1) to give 270 mg of **(21).** 1H-NMR (CDCl3) 5.33 (m, 2H, trans/cis C9,10H, ratio trans/cis 4/96), 2.33 (t, 2H, C2*H*₂) 2.16 ppm (dt, 2H, C17*H*₂) 2.05-1.95 (m,b 4H, C8,11*H*₂), 1.91 (t, 1H, C19*H*), 1.62 (m, 2H, C3*H*₂)-1.50 (m, 2H, C16*H*₂), 1.4-1.2 (m,b 16H, C4-7,12-15*H*₂). 13C-NMR (CDCl₃): 180.19 ppm (C1), 129.87 (C10), 129.79 (C9), 84.63 (C18), 68.04 (C19), 34.04 (C2), 29.66 (C7 + C12), 29.12 (C4 + C14), 29.05 + 29.01 + 28.98 (C4 + C6 + C13), 28.71 (C16), 28.46 (C15), 27.15 + 27.14 (C8 + C11), 24.64 (C3), 18.37 (C17). Pos. ion LC-MS: expected m/z = 310.2746 [M + NH₄⁺].

### 16-Heptadecynoic acid (Alkyne-Palmitate, aPa1) (6)

Synthesis accordingly from methyl-juniperate by PCC-oxidation, Bestmann-Ohira reaction and ester cleavage. 1H-NMR (CDCl₃) 2.32 (t, 2H, C2*H*₂) 2.15 ppm (dt, J=5.5 Hz, 2.6 Hz, 2H, C15*H*₂) 1.91 (t, J=2.6 Hz, 1H, C17*H*), 1.61 (m, 2H, C3*H*₂), 1.50 (m, 2H, C14*H*₂) 1.4-1.2 (m,b 20H, C4-13*H*₂).
Pos. ion LC-MS: expected m/z = 284.2584 [M + NH₄⁺].

### Cell culture

A431 and COS7 cells were maintained in DMEM (Gibco 31966), supplemented with 10% FCS. Huh7 cells were cultured in RPMI (Gibco 31870) with 10% FCS, 0.1 mM non-essential amino acids, 2 mM L-glutamine and 10 mM HEPES. All cells were kept at 37°C and 5% CO₂. Primary hepatocytes were obtained by perfusion of mouse liver with collagenase according to published procedures, and were kept in Williams E medium (Gibco Nr. A12176-01).

### Fatty acid labeling

Cultivated mammalian cells were labeled by supplementation of fatty acids in the respective growth media at final concentrations of 5-50 µM. For labeling of mouse liver, the liver was perfused with Williams E medium supplemented with 10 mg/ml fatty acid-free BSA, 66 µM alkyne-oleate and 33 µM alkyne-palmitate at a flow rate of 5 ml/min. *E. coli* strain BL 21 were grown in minimal medium (M9 supplemented with 0.5 % glucose and 3 mg/l FeSO₄ x 7 H₂O) and incubated for 3h with 20 µM alkyne fatty acids in minimal medium plus 5 mg/ml BSA. *Drosophila melanogaster* L3 larvae were opened by longitudinally cutting the cuticle and incubated in PBS supplemented with 10 mg/ml BSA and 50 µM alkyne fatty acid for 40 min.

### Imaging

A LED narrowband lamp was constructed using commercial 1 W 420 nm LEDs (Roithner Lasertechnik, Vienna, Austria), equipped with a HEBO V01 (Hebo Spezialglas) excitation filter. Images were acquired with a Rolera MGI plus EMCCD camera (Decon Science Tec, Hohengandern, Germany), equipped with a 494/20 and 572/28 bandpass emission filter wheel, all under control of GelPro analyzer (Media Cybernetics) software. Using a macro, at total of 16 images of each plate are taken; with each of the two filters eight images with exposure times between 20 ms and 5 s. The EM gain was set to halve-maximum.

To correct for inhomogeneities in illumination intensity and camera sensitivity, a picture of an empty plate without the emission filter was taken. Using the Fiji software package (www.fiji.sc), fluorescence images were divided through this picture. If necessary, particularly if very small amounts of sample were used, some background bands with broad fluorescence emission can be reduced by subtraction of a picture obtained in the 572 nm (noise) channel from that obtained in the 494 nm (signal) channel. Final quantification was performed using the GelPro analyzer software.

A highly sensitive electron multiplying (EM) CCD camera is employed, to use the imaging system in parallel to low-light applications such as western blot ECL detection. For the present application, in which signal intensity is high, the electron multiplication function would be dispensable. The camera is nonetheless very useful because of its large dynamic range (20,000), which allows taking advantage of the large linear dynamic range of the method (see Fig. 3). The spatial resolution (512x512 pixel) of the camera is fully sufficient for imaging lipid bands on TLC plates.

**Standard procedure,** all values calculated for **3 cm dishes:**
Cells are incubated with alkyne-compounds (typically 5-50 µM alkyne fatty acid) in regular growth medium supplemented with 10 mg/ml fatty acid free BSA and washed twice with 1 ml PBS + 10 mg/ml fatty acid free BSA and once with 1 ml PBS. Cells are scraped into 0.3 ml PBS and transferred into a 2 ml tube. Methanol (0.6 ml) and chloroform (150 µl) are added and the tube is briefly vortexed to obtain a single liquid phase with most of the cellular protein forming a precipitate (if a two-phase mixture forms, add methanol dropwise until a single phase forms). The tube is centrifuged at 14000 x g for 2 min, the supernatant transferred into a fresh tube and the pellet discarded (can be used for analysis of protein if necessary). Chloroform (300 µl) and 1% aqueous acetic acid (600 µl) are added followed by vortexing and centrifugation at 14000 x g for 5 min. The upper aqueous phase is discarded, the lower organic phase transferred into a fresh 1.5 ml reaction vessel (Sarstedt Nr. 72.690.001) and dried in a speed-vac. The dry lipid pellet is redissolved by addition of 7 µl chloroform, followed by addition of 30 µl click reaction mixture (5 µl of 44.5 mM 3-azido-7-hydroxycoumarin, 500 µl of 10 mM [acetonitrile]₄CuBF₄ in acetonitrile, 2 ml ethanol). The tube is incubated in a heating block (Eppendorf Thermomixer comfort, 24 x 1.5 ml block, 42°C, no shaking), until all solvent is condensed under the lid of the tube (typically 3 h). The tube is briefly centrifuged, the lipids re-dissolved by mixing for 1 min at 42°C and applied onto a 20 x 20 cm silica TLC plate (no UV-indicator, Merck No. 1.05721.0001). The plate is developed in CHCl₃/MeOH/water/AcOH 65/25/4/1 for 10 cm, dried for 2 min in a warm stream of air, and developed again for 18 cm in hexane/ethyl acetate 1/1. The plate is briefly dried in a stream of warm air and soaked for 5 sec in 4% N,N-diisopropylethylamine in hexane. The plate is placed in a hood for 1 min to evaporate excess solvent, followed by fluorescent imaging (exc. 420 nm, em. 482-502 nm).

**Variants of the standard procedure:** For **larger dishes,** the lipid extraction procedure is scaled up accordingly. Afterwards, the dried lipid pellet is dissolved and reacted exactly as described above. Rarely, using large dishes and long fatty acid labeling times, the amount of dye in the click mix becomes limiting. In these cases, it is recommended to use only a fraction of the lipid extract for the click reaction or to use smaller dishes from the beginning. For **small dishes,** particularly **96-well** plates, scraping of cells is inefficient and time consuming. Therefore, lipid extraction is performed on the plate. After labeling and washing, the plate is centrifuged upside down for 1 min at 500 g to remove excess liquid. Then, CHCl₃/MeOH 1/5 (100 µl per well) is added and the plate gently agitated for 1 min. This extract is transferred into 1.5 ml tubes, dried in the speed-vac and click-reacted according to the standard protocol.

### Results

### Click fatty acids

Cells contain numerous different fatty acids with different number of carbon atoms and double bonds. Previous papers have described clickable azido- and alkyne-analogues with various chain lengths of saturated fatty acids, recently reviewed in (Hannoush RN and Sun J, 2010. Nat Chem Biol 6, 498-506), of linoleate and arachidonate (Milne SB et al., 2012. Nat Chem Biol 6, 205-207), but not of oleate. For radioisotope tracing of fatty acid metabolism, oleate and palmitate are most frequently used, because they represent the most abundant fatty acids in mammalian circulation and in cellular lipids. Therefore oleate and palmitate analogues containing terminal alkyne groups for click-detection were synthesized.

The synthesis of alkyne-palmitate was achieved by esterification of commercial 16-hydroxy-palmitate, followed by oxidation to the terminal aldehyde and introduction of the triple bond using the Bestmann-Ohira reagent¹². Alkaline cleavage of the methyl ester gave the product in good yields. For the corresponding synthesis of allcyne-oleate, no 18-hydroxy-oleate is commercially available. The compound was therefore synthesized (cf. Figure 6 and Figure 13 and the synthesis protocol described above) starting from 9-decyn-1-ol/9-bromononanoic acid or nonandiole. In case of nonandiole the diole was protected and oxidized to obtain intermediates that were coupled by a Wittig reaction to obtain the key intermediate (17), a protected hydroxy oleate, with a cis/trans ratio of 78/22. By treatment with MeOH/H⁺, this was converted into the methyl ester of hydroxy-oleate **(18).** To improve the cis/trans ratio, the intermediate **(18)** was purified by argentation chromatography to give a satisfactory cis/trans ratio of 97/3. The product was then oxidized **(19)** and treated with Bestmann-Ohira reagent as above to give the alkyne **(20),** followed by cleavage of the methyl ester to give alkyne-oleate **(21).**

### Detection strategy and click reaction

Detection of substances on TLC plates is often achieved by treatment with spray reagents resulting in colored spots. This procedure lacks control over the sprayed amounts and leads to problems in quantification. Furthermore, the complex nature of the click reaction appears to be not compatible with an on-plate reaction. Therefore, the detection reaction was performed before the TLC separation, using 3-azido-7-hydroxycoumarin, a small, non-polar detection dye, which would not dominate the migration behavior of the reaction products on the TLC plate. In addition, 3-azido-7-hydroxycoumarin is a non-fluorescent compound that forms a fluorescent azido-coumarin derivative only upon click reaction with the alkyne fatty acid (Figure 1). This fluorogenic reaction avoids the problem of strong background signal from excess of unreacted dye.

The click reaction was optimized to detect the very small amounts of labeled lipids that are obtained in a typical labeling experiment. Cells were labeled in a 15 cm dish for 2h with a 1/1 mixture of alkyne-oleate/alkyne-palmitate (100 µM total) and dissolved the extracted lipids in 1 ml of chloroform. For initial reactions, 20 µl aliquots of this extract were evaporated, re-dissolved in 50 µl ethanol and reacted for 2 h with various concentrations of 3-azido-7-hydroxycoumarin, copper(I) and the copper(I)-stabilizer TBTA (Chan TR et al., 2004. Organic Letters 6, 2853-2855). As a source of copper(I) ions the complex [acetonitrile]₄CuBF₄ was used, which is stable in acetonitrile stock solution over months and readily soluble in ethanol. The presence of TBTA accelerated the click-reaction, but also led to the appearance of a strongly fluorescent background band that disturbed the analysis (data not shown). It was therefore omitted in further experiments. Further improvement was achieved by reducing the reaction volume, leading to a virtually complete reaction for analyte concentrations up to at least 15 µM (Figure 2a). Interestingly, temperature control and the geometry of the reaction step was of prime importance. When a 37µl sample was incubated in a standard heating block, the solvent evaporated within 2.5-3 h and condensed under the lid. When the same tube was placed in an incubator, the liquid did not evaporate and condense, and the yield of the reaction was reduced to 30-40%. It can be concluded that the concentration achieved by evaporation drives the reaction to completion. The importance of the dye concentration is illustrated in Figure 2b. Additional dye did not lead to stronger signal but only to increased background.

### Separation

After the click reaction, the entire reaction volume was applied onto standard silica gel TLC plates, which were developed first for about 10 cm in CHCl₃/MeOH/H₂O/AcOH 65/25/4/1 to separate the polar lipids, followed by a second run in hexane/ethyl acetate 1/1 to separate neutral lipids. Qualitatively, the clicked lipids behaved very similar to their natural counterparts, i.e. the normal order of mobility is unchanged (Figure 2b). Quantitatively, the clicked polar lipids migrated very similar to the natural lipids, while the clicked neutral lipids ran significantly slower, which was compensated by a slightly higher polarity of the second solvent system (we normally use hexane/ethyl acetate 4/1 for natural lipids).

### Detection and quantification

In the click reaction, a 3-triazolyl-7-hydroxycoumarin is formed, which exists, depending on the pH and the nature of the solvent, either in the protonated hydroxy-form or in the deprotonated phenolate-form of the molecule. For quantitative analysis it is necessary that all molecules are either in the hydroxy- or the phenolate-form, because they absorb and fluoresce at different excitation and emission wavelengths (OH-form: Exc. 354 nm, Em. 423 nm; O⁻-form: Exc. 412 nm, Em. 484 nm, measured for the product of the click reaction between 3-azido-7-hydroxycoumarin and alkyne-palmitate in ethanolic solution). It was decided to image the phenolate form, because excitation at 420 nm lead to reduced background compared to excitation at 360 nm (data not shown). On the slightly acidic silica gel most of the hydroxycoumarin derivative will be in the protonated form, supported by the acidic first solvent, which is necessary to separate PA from PC. Therefore, the dried plate was soaked for few seconds in a solution of N,N-diisopropylethylamine (Hünig's base), a volatile non-fluorescent basic reagent, in hexane. Under these conditions, the hydroxycoumarin derivative is converted completely to the O⁻-form (data not shown), resulting in a striking 60-fold increase of the signal (Figure 2b and c).

### Sensitivity and linearity

Sensitivity and linear dynamic range of the assay was determined using the cholesterol ester of alkyne-oleate as a model substance. With a reaction volume of 30 µl, the lower detection level was at a concentration of 13.5 nM corresponding to 0.4 pmol total substance (Figure 3a). Over a concentration range from 13.5 nM to 40 µM, the response curve was nearly linear, as shown by the slope of 0.91 in a double logarithmic plot (Figure 3b). A similar experiment with more data points in a more narrow range revealed good linearity over the practically most important concentration range between 0.5 and 15 µM (Figure 3c).

### Lipid standards

An obvious problem of the pre-separation derivatization is the requirement of authentic click-labeled co-migrating lipid standards for identification of labeled spots. For that, TAG, 1,2-DAG, 1,3-DAG, 1-MAG, CE, PC and PA were chemically synthesized, all bearing alkyne-oleate. A PE standard was obtained by preparative TLC separation of lipids from E. coli grown in medium supplemented with alkyne-oleate. Figure 7 shows the purity of the standards and also demonstrates the good separation in the two-solvent TLC system.

### Metabolism of alkyne fatty acids

In order to use alkyne-fatty acids as tracers for natural fatty acids, it is necessary to study similarities and differences in their metabolism. To compare the metabolism of alkyne-oleate and natural oleate, freshly isolated hepatocytes were incubated with equal concentration of alkyne-oleate and oleate plus a tracer amount of ³H-oleate. After 10 min labeling and a 20 min chase, lipids were extracted, split into equal aliquots and analyzed either by the standard click procedure or by TLC, fluorography, scraping and scintillation counting. The comparison between the fluorography and the click-image (Figure 4a) showed that qualitatively both methods gave similar results, with most of the label found in PC, PE and neutral lipids (NL). Regarding the sensitivity of the methods, the fluorography showed, despite a 5 day exposure, clearly visible bands only for the three most abundant products. In contrast, a 2 s exposure of the clicked lipids reveals a clear picture with much stronger signal and less background, showing several additional weak bands of minor metabolites. For a quantitative evaluation, the click-image was directly quantified, while radioactive bands were scraped, lipids extracted and subjected to scintillation counting. This is necessary because the fluorography on X-ray film is essentially nonlinear and over-represents strong signals relative to weak ones, which can only be partially compensated by pre-flash procedures¹⁶. Comparing the three major products, the amount of label in neutral lipids was slightly smaller for alkyne-oleated (49%) than for ³H-oleate (64%); the difference was proportionally distributed over PC and PE. For a closer inspection of the distribution of labeled species, the analysis was extended to alkyne-palmitate. Freshly isolated mouse hepatocytes were labeled for various time periods with either alkyne-oleate or alkyne-palmitate (Figure 8a, lanes 1-6). Control cells were incubated for the same time periods with natural oleate or palmitate (Figure 8a, lanes 7-12). Cells were harvested and lipids analyzed by fluorescent click reaction or by mass spectrometry. In fluorescent analysis, a time dependent increase of signal was observed, with strong bands representing PC, PE, free FA, DAG and TAG. At all labeling times, there was a strong preference of alkyne-oleate for incorporation into TAG compared to PC, while alkyne-palmitate showed an equal distribution between PC and TAG (Figure 8b). The incubation of cells with oleate, palmitate or the two alkyne fatty acids for 10-120 min did not influence the species composition of PC, PA, PE, and PG (Figures 8 and 9a and b).

### Typical applications

In the following section, possible applications are demonstrated in a broad variety of biological systems and sample sizes. Although some of the results are interesting per se, the focus is on the adaptability and convenience of general use. An important future application of the system is pulse-chase analysis of fatty acid metabolism in cell lines. HuH7 hepatoma cells were pulse-labeled for 3 min with either alkyne-palmitate (Figure 5a, aPal, lane 4) or alkyne-oleate (Figure 5a, aOle, lane 6). Apart from some PC and TAG, much of the fatty acids were found as the free substance, or in biosynthetic intermediates such as PA and DAG. Upon a 30 min chase, labeled precursors and intermediates in the fatty acid labeled samples were metabolized to the final products PC, PE, TAG and CE (lanes 5 and 7).

For high-throughput applications, miniaturization of the procedure is necessary. Figure 9 demonstrates that the protocol can be adapted to 96-well plates. In this format, scraping of cells is very tedious and was replaced by direct solvent extraction of lipids on the dish. For that, either CHCl₃/MeOH 1/5 was used, which does not dissolve the plastic surface, followed by drying and standard click reaction (sequential procedure), or the click reaction mixture for lipid extraction was directly used (one-step procedure). To compensate for the small surface of a 96-well (1/29 of a 6-well), a longer labeling time of 75 min with reduced fatty acid concentrations of 5-20 µM was selected. Although qualitatively similar, closer inspection revealed that the sequential procedure (Figure 9a) gave double the signal intensity and contained more information about low abundance lipid classes. Due to the low amounts of labeled lipids, the background of the entire procedure (see lanes 7, 8 in all panels) was more pronounced than in other applications.

In the final example (Figure 5c), the use of the method to follow lipid metabolism in systems other than mammalian cell culture was explored. A mouse liver was perfused for 5 min with 100 µM of a 2/1 mixture of alkyne-oleate and alkyne-palmitate, reflecting typical ratio of plasma oleate and palmitate. After a short wash, the tissue was homogenized, lipids extracted and a small aliquot analyzed using the standard protocol resulting in strong labeling of both phospholipids and neutral lipids (Figure 5c, lane 1). At the other end of the phylogenetic tree, *E. coli* bacteria were incubated in minimal medium with either of the two alkyne fatty acids. Lipid extracts revealed incorporation into PE and PG (Fig. 5c, lanes 2+3) at a ratio around 4/1 (for detailed quantification see Figure 5d), consistent with previous determinations of *E. coli* phospholipid class distribution. Incorporation of alkyne-oleate into lipids of the yeast *Saccharomyces cerevisiae,* one of the classical model systems of lipidology, resulted in strong labeling of phospholipids and TAG (Figure 5c, lane 6).

*Drosophila melanogaster* is a powerful genetic model system with increasing importance for lipid research. To demonstrate fatty acid metabolism in *Drosophila* tissues, the cuticle of single L3 larvae were opened and incubated them for 30 min in the respective alkyne fatty acid solution. Lanes 4 and 5 demonstrate incorporation into PC, PE and TAGs. There was a large difference between metabolism of the two alkyne fatty acids: while similar amounts of both fatty acids were found in PC, large amounts of alkyne-oleate but much less alkyne-palmitate was incorporated into PE. This reflects both the total phospholipids class distribution and the fatty acid composition of *Drosophila* membrane lipids.

All documents cited herein, are hereby incorporated by reference in their entirety.

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. In addition, where features or aspects of the invention are described in terms of Markush groups, those skilled in the art will recognize that the invention is also thereby described in terms of any individual member or subgroup of members of the Markush group. Further embodiments of the invention will become apparent from the following claims.

## Claims

1. Method for the detection of a lipid comprising:
a) reacting in a solvent at least one lipid derivatized with at least one terminal alkyne group with at least one coumarin derivative compound derivatized with at least one terminal azido group in the presence of catalytic amounts of Cu(I) to form a triazolyl coumarin compound derivative of the at least one lipid,
b) purifying the triazolyl coumarin compound derivative of the at least one lipid by chromatography, and
c) detecting the triazolyl coumarin compound derivative of the at least one lipid,
wherein
i) after purifying of the triazolyl coumarin compound derivative of the at least one lipid and before its detection the triazolyl coumarin compound derivative of the at least one lipid the compound is treated with a base; and/or
ii) the reacting step comprises providing the at least one coumarin derivative compound derivatized with at least one terminal azido group in excess over the at least one lipid derivatized with at least one terminal alkyne group and evaporating the solvent to concentrate the reaction mix and achieve complete reaction of the at least one lipid derivatized with at least one terminal alkyne group with the at least one coumarin derivative compound derivatized with at least one terminal azido group to form a triazolyl coumarin compound derivative of the at least one lipid.

2. The method of claim 1, wherein
i) the lipid of the lipid derivative is selected from the group consisting of saturated fatty acids, unsaturated fatty acids, glycerolipids, glycerophospholipids, lysophosphoglycerolipids, sphingolipids, sterol lipids, prenol lipids, saccharolipids, carotenoids, waxes, and polyketides,
ii) the azido coumarin derivative is a 3-azido hydroxy coumarin,
iii) the coumarin derivative is selected from the group consisting of 3-, 4-, 5-, 6-, 7-, and 8-hydroxy azido coumarin,
iv) reaction step a) comprises the addition of a Cu(I) salt or complex,
v) reaction step a) comprises the addition of a Cu(I) salt or complex, wherein the Cu(I) salt or complex is selected from the group consisting of CuI, CuBr, CuCl, CuOTf*C₆H₆, [Cu(NCCH₃)₄], and Cu[acetonitrile]₄BF₄, Cu[acetonitrile]₄PF₆, and combinations thereof,
vi) reaction step a) comprises evaporating the solvent of the reaction mixture to increase the concentration of the at least one lipid derivatized with at least one terminal alkyne group to a concentration range of 5-40 µM,
vii) reaction step a) comprises evaporating the solvent of the reaction mixture to increase the concentration of the at least one coumarin derivative compound derivatized with at least one terminal azido group to a concentration range of 50-5000 µM, optionally in the range of 300-1000 µM,
viii) reaction step a) comprises providing the at least one coumarin derivative compound derivatized with at least one terminal azido group with a concentration range of 40-100 µM, optionally 60 µM,
ix) the chromatography of step b) is selected from the group consisting of HPLC, gel chromatography, TLC, and SMART,
x) the chromatography of step b) is TLC and the samples are first developed in CHCl₃/MeOH/H₂O/AcOH 65/25/4/1 and then in hexane/ethyl acetate 1/1
xi) the detection in step c) is fluorescence detection;
xii) the base of step i) is selected from the group of volatile and non-fluorescent bases, and/or
xiii) the base of step i) is selected from the group of bases consisting of N,N-diisopropylethylamine, triethylamine, trimethylamine, monoethylamine (MEA), diethylamine (DEA), triethylamine (TEA), monoisopropylamine, monoisopropylamine (MIPA), diisopropylamine (DIPA), dimethylaminopropylamine (DEAPA), dimethyl dipropylene Triamine (DMAPAPA), 3-Isopropoxypropylamine (IPOPA), 3-methoxypropylamine (MOPA), ethylmethylamine (EMA), tetramethylpropylenediamine (TMPDA), sec-butylamine (B2A), aminopropyldiethanolamine (APDEA), dimethyl isopropyl amine (DMIPA), dimethylethylamine (DMEA), and diethylhydroxylamine (DEHA), and combinations thereof.

3. The method according to claims 1 and 2, wherein
i) the lipid of the lipid derivative is selected from the group consisting of oleate, palmitate, cholesterol, cholesterol ester, cardiolipin, diacylglycerol, phosphatidylcholine, phosphatidylethanolamine, phosphatidylglycerol, phosphatidylinositol, phosphatidylserine, triacylglycerol, and sphinganin, and/or
ii) the method comprises extracting the at least one lipid derivatized with at least one terminal alkyne group from a cell prior to step a), and/or
iii) the method comprises, prior to step a), extracting the at least one lipid derivatized with at least one terminal alkyne group from a cell which has been incubated with at least one lipid derivatized with at least one terminal alkyne group, and/or
iv) the method comprises, prior to step a), extracting the at least one lipid derivatized with at least one terminal alkyne group from a cell which has been incubated with at least one lipid derivatized with at least one terminal alkyne selected from the group consisting of terminal alkyne cholesterol derivative, omega alkyne oleate, omega alkyne palmitate, omega alkyne sphinganin, omega alkyne fatty acid, and omega alkyne unsaturated fatty acid.

4. A compound having the structural formula: 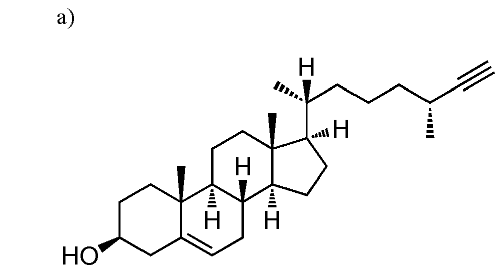 or a salt and/or ester thereof, 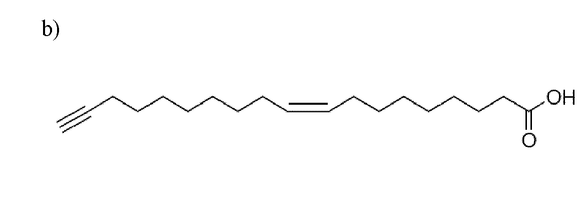 or a salt and/or ester thereof, or 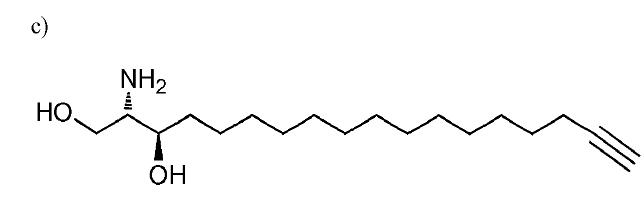 or a salt and/or ester thereof.

5. A fluorescent adduct obtained by reacting a compound according to claim 4 with a 3-, 4-, 5-, 6-, 7-, or 8-hydroxy azido coumarin.

6. A fluorescent adduct obtained by reacting a compound according to claim 4 with an azido moiety bound to a fluorophore.

7. Use of a compound according to claims 5-6 in a fluorescence assay.

8. Use of a compound according to claims 5-6 for determining an enzyme activity.

9. Use of a compound according to claims 4-6
a) for analyzing or monitoring the lipid metabolism in a cell,
b) for synthesis of alkyne lipid derivatives.

10. Method of synthesis of alkyne lipids derivatives comprising:
contacting a cell with at least one compound selected from the group consisting of compounds according to claim 4 and alkyne palmitate,
cultivating the cell under conditions wherein the at least one compound selected from the group consisting of compounds according to claim 4 and alkyne palmitate is subjected to the cell's lipid metabolism, and
extracting the alkyne derivatives produced by the cell.

11. The method of claim 10, wherein the synthesized alkyne lipid derivatives are detected using the method according to claims 1-3.

12. Method of synthesis of (2S,3R)-2-aminooctadec-17-yn-1,3-diol (alkyne-sphinganine) comprising:
a) reacting THP-protected 1-tetradecyn-14-ol and Garners aldehyde to give *tert-Butyl* (4*S*,1'*R*)-4-(1'-hydroxypentadec-2'-yn-15-(2-tetrahydropyranyloxy)-yl-2,2-dimethyl-3-oxazolinecarboxylate,
b) reacting the product of step a) with H₂ in the presence of Pd/C to give *tert*-Butyl (4S,1'*R*)-4-(1'-Hydroxypentadec-15-(2-tetrahydropyranyloxy)-yl-2,2-dimethyl-3-oxazolinecarboxylate,
c) reacting the product of step b) with acetic anhydride in the presence of pyridine and DMAP to give *tert-Butyl* (4*S*,1'*R*)-4-(1'-Acetoxypentadec-15-(2-tetrahydropyranyloxy))-yl-2,2-dimethyl-3-oxazolinecarboxylate,
d) reacting the product of step c) with acetone, MeOH, and toluenesulfonic acid to give *tert-Butyl* (4*S*,1*'R*)-4-(1'-Acetoxypentadec-15-hydroxy)-yl-2,2-dimethyl-3-oxazolinecarboxylate,
e) reacting the product of step d) with pyridiniumchlorochromate (PCC) in the presence of powdered molecular sieves to give the corresponding aldehyde,
f) reacting the product of step e) with dimethyl (1-diazo-2-oxopropyl)phosphonate (Bestmann-Ohira reagent), potassium carbonate, and MeOH to give *tert*-Butyl (4*S*,1'*R*)-4-(1'-hydroxyhexadec-15-yn)-yl-2,2-dimethyl-3-oxazolinecarboxylate, and
g) reacting the product of step f) in THF with HCl to give (2S,3R)-2-Aminooctadec-17-yn-1,3-diol.

13. Method of synthesis of (25*R*)-25-ethinyl-26-nor-3*b*-hydroxycholest-5-en (click-Cholesterol) comprising:
a) reacting (25R)-3b-(tert-Butyldimethylsilyloxy)cholest-5-en-26-ol with pyridiniumchlorochromate (PCC) in the presence of powdered molecular sieves to give (25*R*)-3*b*-(*tert*-Butyldimethylsilyloxy)cholest-5-en-26-al, and
b) reacting the product of step a) with dimethyl-(1-diazo-2-oxopropyl)phosphonate (Bestmann-Ohira reagent), potassium carbonate, MeOH, and THF, followed by the treatment of the resulting product with HCl and THF to give (25*R*)-25-ethinyl-26-nor-3b-hydroxycholest-5-en.

14. Method of synthesis of a compound having a structural formula CH≡C-(CH₂)ₙ-COOH, wherein n is an integer selected from the group consisting of 1, 2, 3, 4, 5, 6, 7, 8, 9, 10, 11, 12, 13, 14, 15, 16, 17, 18, 19, 20, 21, and 22, comprising:
a) reacting a compound having a formula Br-CH₂-CH₂-(CH₂)ₙ-COOH with KOH to give HO-CH₂-(CH₂)ₙ-COOH,
b) reacting the product of step a) with ROH, H⁺ to give HO-CH₂-(CH₂)ₙ-COO-R, wherein R is selected from the group comprising -CH₃, -CH₂CH₃, -n-propyl, -i-propyl, -n-butyl, -sec-butyl, and -t-butyl,
c) reacting the compound of step b) with pyridinium chlorochromate (PCC) in the presence of powdered molecular sieves to give O=CH-CH₂-(CH₂)ₙ-COO-R,
d) reacting the compound of step c) with potassium carbonate, dimethyl (1-diazo-2-oxopropyl)phosphonate (Bestmann-Ohira reagent), and methanol to give CH≡C-CH₂-(CH₂)ₙ-COO-R, and
e) subjecting the product of step d) to ester hydrolysis to give CH≡C-CH₂-(CH₂)ₙ-COOH.

15. Method of synthesis of omega alkyne unsaturated fatty acids comprising:
I)
a) reacting a compound having a formula of HC≡C-(CH₂)ₙ-CH₂-OH with pyridinium chlorochromate (PCC) in the presence of powdered molecular sieves to give HC=C-(CH₂)ₙ-CH=O, wherein n is an integer selected from the group consisting of 1, 2, 3, 4, 5, 6, 7, 8, 9, 10, and 11,
b) reacting a compound having a formula of Br-CH₂-(CH₂)ₘ-CH₂-OH with CrO₃/H₂SO₄ to give Br-CH₂-(CH₂)ₘ-COOH, wherein m is an integer selected from the group consisting of 1, 2, 3, 4, 5, 6, 7, 8, 9, and 10,
c) reacting Br-CH₂-(CH₂)ₘ-COOH with triphenylphosphin at a temperature of 80-150°C to give the corresponding bromine salt of the omega triphenylphosphinyl fatty acid derivative, and
d) reacting the product of step c) with two equivalents of lithium hexamethylene-disilazane in dry THF under argon, cooling the reaction mix to -55°C, adding 1,3-dimethyl-3,4,5,6-tetrahydro-2(1H)-pyrimidinone (DMPU), followed by addition of the product of step a) solubilized in THF to give HC≡C-(CH₂)ₙ-CH=CH-(CH₂)ₘ-COOH;
or
II)
a) reacting a compound having a formula of HO-CH₂-(CH₂)ₙ-CH₂-OH with propionic acid and toluenesulfonic acid to give CH₃C(=O)O-CH₂-(CH₂)ₙ-CH₂-OH, , wherein n is an integer selected from the group consisting of 1, 2, 3, 4, 5, 6, 7, 8, 9, 10, 11, and 12
b) reacting the compound of step a) with pyridinium chlorochromate (PCC) in the presence of powdered molecular sieves to give CH₃C(=O)O-CH₂-(CH₂)ₙ-CH=O,
c) reacting a compound having a formula of Br-CH₂-(CH₂)ₘ-CH₂-OH with CrO₃/H₂SO₄ to give Br-CH₂-(CH₂)ₘ-COOH, wherein m is an integer selected from the group consisting of 1, 2, 3, 4, 5, 6, 7, 8, 9, 10, 11, and 12,
d) reacting Br-CH₂-(CH₂)ₘ-COOH with triphenylphosphin at a temperature of 80-150°C to give the corresponding bromine salt of the omega triphenylphosphinyl fatty acid derivative,
e) reacting the product of step b) in dry THF under argon with two equivalents of lithium hexamethylene-disilazane, cooling the reaction mix to -55°C, adding 1,3-dimethyl-3,4,5,6-tetrahydro-2(1H)-pyrimidinone (DMPU), followed by addition of the product of step b) solubilized in THF to give CH₃C(=O)O-CH₂-(CH₂)ₙ-CH=CH-(CH₂)ₘ-COOH,
f) reacting the product of step e) with MeOH and acetylchloride to give *cis* HO-CH₂-(CH₂)ₙ-CH=CH-(CH₂)ₘ-COO-CH₃,
g) reacting the compound of step f) with pyridinium chlorochromate (PCC) in the presence of powdered molecular sieves to give *cis* O=CH-(CH₂)ₙ-CH=CH-(CH₂)ₘ-COO-CH₃,
h) reacting the compound of step g) with potassium carbonate, dimethyl (1-diazo-2-oxopropyl)phosphonate (Bestmann-Ohira reagent), and methanol to give *cis* CH=C-(CH₂)ₙ-CH=CH-(CH₂)ₘ-COO-CH₃, and
i) reacting the product of step h) with KOH, MeOH, H₂O and HCl to give *cis* CH=C-(CH₂)ₙ-CH =CH-(CH₂)ₘ-COOH.
